(19) **Europäisches Patentamt**

**European Patent Office**

**Office européen des brevets**

(11) **EP 1 211 511 B1**

(12) **EUROPÄISCHE PATENTSCHRIFT**

(45) Veröffentlichungstag und Bekanntmachung des Hinweises auf die Patenterteilung:
**14.07.2004 Patentblatt 2004/29**

(51) Int Cl.⁷: **G01N 33/38**, G01N 3/32, G01N 29/12

(21) Anmeldenummer: **01126236.7**

(22) Anmeldetag: **05.11.2001**

(54) **Verfahren zur zerstörungsfreien, automatisierten Festigkeitsbestimmung an Prüfkörpern sowie Prüfvorrichtung zur zerstörungsfreien Festigkeitsbestimmung**

Method for non-destructive, automated determination of the strength of test pieces, and test apparatus for non-destructive strength determination

Méthode non-destructive et automatique pour déterminer la résistance mécanique de pièces d'essai, et appareil de test pour la détermination non-destructive de la résistance mécanique

(84) Benannte Vertragsstaaten:
**AT BE CH CY DE DK ES FI FR GB GR IE IT LI LU MC NL PT SE TR**

(30) Priorität: **07.11.2000 DE 10055099**

(43) Veröffentlichungstag der Anmeldung:
**05.06.2002 Patentblatt 2002/23**

(73) Patentinhaber: **DYCKERHOFF AKTIENGESELLSCHAFT**
**D-65203 Wiesbaden (DE)**

(72) Erfinder:
• **Sievert, Thomas**
**55276 Oppenheim (DE)**
• **Wolter, Albrecht, Prof. Dr.-Ing.**
**38678 Clausthal-Zellerfeld (DE)**

(74) Vertreter: **Solf, Alexander, Dr.**
**Patentanwälte**
**Dr. Solf & Zapf**
**Candidplatz 15**
**81543 München (DE)**

(56) Entgegenhaltungen:
**EP-A- 0 343 033**

• **DIN: "EN 196-1" 1994 XP002207952 * Absatz [08.3] - Absatz [08.4] ***
• **ASTM: "C215-91" 1991 XP002208004 * Absatz [04.3] * * Absatz [06.2] * * Absatz [07.1] - Absatz [07.3] * * Absatz [09.1] - Absatz [09.4] ***
• **ASTM: "C192/C192M-95" 1995 XP002208005 * das ganze Dokument ***
• **ROEBBEN G ET AL: "IMPULSE EXCITATION APPARATUS TO MEASURE RESONANT FREQUENCIES, ELASTIC MODULI, AND INTERNAL FRICTION AT ROOM AND HIGH TEMPERATURE" REVIEW OF SCIENTIFIC INSTRUMENTS, AMERICAN INSTITUTE OF PHYSICS. NEW YORK, US, Bd. 68, Nr. 12, 1. Dezember 1997 (1997-12-01), Seiten 4511-4515, XP000726321 ISSN: 0034-6748**
• **PATENT ABSTRACTS OF JAPAN vol. 1998, no. 01, 30. Januar 1998 (1998-01-30) & JP 09 229836 A (SHIMADZU CORP), 5. September 1997 (1997-09-05)**

**Beschreibung**

Die Erfindung betrifft ein verbessertes, insbesondere automatisiertes Verfahren zur zerstörungsfreien Festigkeitsbestimmung an Prüfkörpern sowie eine Prüfvorrichtung zur zerstörungsfreien Festigkeitsbestimmung.

Ein derartiges zerstörungsfreies Prüfverfahren ist in ASTM 'C215-91' 1991 XP 002207953 beschrieben. Dieses Verfahren beschreibt die Aufnahme der Transversalwelle, der Longitudinalwelle und der Torsionsschwingungsfrequenz zur Kalkulation des E-Moduls, des Torsionsmoduls und Querkontraktionskoeffizienten. Für die Ermittlung der Druckfestigkeiten von Prüfkörpern in Form von Mörtelprismen ist derzeit die zerstörende Prüfung nach DIN EN 196 Teil 1 vorgeschrieben. Zur Ermittlung der Druckfestigkeit müssen für jedes Prüfalter jeweils drei Prismen hergestellt werden. Im Stand der Technik lassen sich immer wieder Werke finden, in denen versucht wird, die Druckfestigkeit zerstörungsfrei über die Ultraschallmethode zu ermitteln. Je nach Ultraschallmethode sind die Einflussfaktoren und damit verbunden die Korrelationen des dynamischen Elastizitätsmoduls und der Druckfestigkeit unterschiedlich stark ausgeprägt. Ein bereits umgesetztes Verfahren der Fa. Geotron arbeitet über die Messung der Schalllaufzeit und erreicht laut Herstellerangaben Abweichungen zur zerstörend ermittelten Druckfestigkeit bei Betonen kleiner als 10 %.

Für die Umsetzung einer solchen Problemstellung sind nachstehende Probleme hinsichtlich der Automatisierbarkeit zu lösen:

- Probenkennzeichnung und Identifikation

- Bestimmung der Prüfkörpermaße zur Volumenbestimmung

- Massebestimmung zur Ermittlung der Prüfrohdichte

- Korrelationsuntersuchungen zwischen der Resonanzfrequenz und der Druck- und Biegezugfestigkeit

Ein wesentlicher Aspekt der Erfindung ist die verbesserte Korrelation zwischen der Resonanzfrequenz und der Biegezug und Druckfestigkeit. Bei der Erarbeitung der formelmäßigen Abhängigkeiten wurde weiterhin überprüft, zu welchem Zeitpunkt nach Herstellung der Prismen mit den Messungen begonnen werden kann. Des weiteren ist auf die Möglichkeiten der Probenkennzeichnung und Identifikation stärker eingegangen worden.

Aufgabe der Erfindung ist es, ein Verfahren und eine Vorrichtung für die zerstörungsfreie Prüfung der Druckfestigkeit mit Hilfe von Ultraschall zu schaffen, wobei eine genauere Aussage über die Festigkeit und ein hoher Automatisierungsgrad bei hoher Prozeßsicherheit erzielt wird.

Die Aufgabe wird mit Verfahren mit den Merkmalen der Ansprüche 1 und einer Vorrichtung mit den Merkmalen des Anspruch 20 gelöst.

Vorteilhafte Weiterbildungen sind in Unteransprüchen gekennzeichnet.

Erfindungsgemäß wird die Druckfestigkeit an Mörtelprismen mit Hilfe einer verbesserten, genaueren Ultraschallprüfung zerstörungsfrei ermittelt, wobei bei einem hohen Automatisierungsgrad des Prüfverfahrens eine eindeutige Probenkennzeichnung und Identifikation sichergestellt wird. Dies geschieht insbesondere mit Hilfe von RFID-Systemen und über die Lage der Prismen im Wasserbecken.

Die Einlagerung der Prüfkörper erfolgt über ein Robotersystem, welches auf einer Linearachse die Front der Wasserbecken abfahren kann; mit Hilfe des Greifarms des Roboters kann die Breite (y-Achse) und Tiefe (z-Achse) der Wasserbecken erreicht werden. Nach der Einlagerung sind die Koordinaten des Lagerplatzes (x und y) mit der Probenummer zur späteren Identifikation gespeichert.

Zum Zeitpunkt der Auslagerung erhält der Roboter über den Steuerungsrechner einen Befehl und fährt zu den entsprechenden Koordinaten des zu prüfenden Prismas. Beispielsweise über eine am Arm des Roboters angebrachte Antenne werden die am Prisma befestigten Datenträger unter Wasser ausgelesen und eindeutig identifiziert. Die für die späteren Bestimmungen nötige Gewichtskonstanz kann über das Abtropfen des Wasser oder über einen Druckluftstoß erreicht werden. Nach Erreichen der Gewichtskonstanz gelangen die Prismen zu den jeweiligen Messstellen.

Die erstellten Korrelationen bestehen zwischen dem dynamischen Elastizitätsmodul, welches aus der Resonanzfrequenz, der Prüfrohdichte und den Abmessungen berechnet wird, und der zerstörend bestimmten Druckfestigkeit. Die Prüfrohdichte und die Abmessungen der Prismen müssen zuvor bestimmt werden. Die Prüfstandsapparatur ist mit Messstellen zur Bestimmung der Masse und der Prüfkörperdimensionen auszustatten. Die Massebestimmung erfolgt über eine Waage mit einer Genauigkeit von 0,1 g. Die Bestimmung der Prüfkörperabmessungen kann sowohl mit Hilfe eines Lasersystems als auch über induktive Wegaufnehmer erfolgen. Die Genauigkeit der beiden Verfahren erreicht ± 0,01 mm und erlaubt auch die Verfolgung der Volumendehnung. Die Ergebnisse werden über eine RS 232 Schnittstelle an eine EDV gesendet und der jeweiligen Probenummer und Prüfzeit zugeordnet.

Nach der Bestimmung der Masse und der Abmessungen erfolgt die Bestimmung der Grundresonanzfrequenzen. Obwohl zur Berechnung des Elastizitätsmoduls nur die Dehnungsresonanz benötigt wird, wird zusätzlich die Torsionsfrequenz zur Kontrolle der Messung ermittelt. Bei der Ankopplung der Prüfköpfe wird ein Ankopplungsdruck vorzugsweise von ca. 1,0 N/mm² vorgesehen. Mit Hilfe der bestimmten Daten kann in Abhängigkeit des Zementes die Druckfestigkeit berechnet werden. Nach der Bestimmung der Grundresonanzen erfolgt die Einlagerung der Prismen auf die festgelegten Lagerplätze.

Aufgrund des zerstörungsfreien Prüfverfahrens kann die Anzahl der Prüfkörper über den Gesamtzeitraum deutlich gesenkt werden. Des weiteren kann beliebig oft gemessen werden, wodurch die Möglichkeit besteht, die Festigkeitsverläufe genauer abzubilden.

Ein erfindungsgemäßer Prüfstand, bietet eine Reihe von Vorteilen:

• Reduzierung der Prismenanzahl durch zerstörungsfreie Messungen (ein Satz für den gesamten Prüfzeitraum)

• Reduzierung des Materialaufwandes (vor allem Normensand)

• Reduzierung des Zeit- und Arbeitsaufwandes

• Reduzierung der notwendigen Kapazität an Klimakammern und Wasserbecken

• Messungen zu beliebigen Zeitpunkten

• Messungen an mehreren Prüfterminen

• Geringere Schwankungen durch Messung an denselben Prismen über den Prüfzeitraum

• Möglichkeit der Dokumentation eines kontinuierlichen Festigkeitsverlaufes

• Möglichkeit zur frühen Abschätzung der 28-Tage-Festigkeiten

• Ersatz der Graf-Kaufmann-Methode durch genauere Volumenbestimmung

• Bessere Überprüfbarkeit der ermittelten Daten durch die zusätzliche Bestimmung der Torsionsfrequenz

Dieses Verfahren kann in allen Bereichen in denen Prüfkörper entsprechend dem obigen Verfahren geprüft werden sollen angewandt werden, insbesondere auch im Bereich der Steine- und Erden-Industrie wie Beton, Kalk und Gips bzw. Bindemittel sowie Rohstoffen unabhängig von der Korngrösse insbesondere der Zuschläge im weitesten Sinne angewendet werden.

Die Erfindung wird Beispielhaft anhand einer Zeichnung erläutert, es zeigen dabei:

Fig. 1     Ein Lesesystem für Prüfkörper mit passivem Datenträger;

Fig. 2     Abtropfkurven von unter Wasser gelagerten Prüfkörpern;

Fig. 3     einen Prüfkörper mit angeordneten Messköpfen in einer schematischen Seitenansicht;

Fig. 4     eine graphische Darstellung des Mittelwertes als Funktion der Anzahl der durchgeführten Messungen;

Fig. 5     den Verlauf der Verstärkung und der Resonanzfrequenz in Abhängigkeit von der Anpresskraft;

Fig. 6     das Verhältnis der Grundresonanzen zueinander in Abhängigkeit vom Probenalter;

Fig. 7     die Korrelation des dynamischen E-Moduls und der Druckfestigkeit;

Fig. 8     die Fittfunktion;

Fig. 9     die Korrelation zwischen zerstörungsfrei und zerstörend bestimmter Druck- und Biegezugfestigkeit;

Fig.10     die Übersicht über die Bestimmtheitsmasse;

Fig.11 den Faktor a als Funktion des Lageparameters x';

Fig.12 die Korrelation des berechneten Faktors mit dem bestimmten Faktor a;

Fig.13 den Exponenten b als Funktion des Faktors a;

Fig.14 die Korrelation zwischen dem berechneten Exponenten und dem bestimmten Exponenten;

Fig.15 die erfindungsgemässe Prüfstandsapparatur schematisch als Blockschaltbild;

Fig.16 das Robotersystem und die Anordnung der Messstellen in einer schematischen Darstellung;

Fig.17 die Einspannvorrichtung zur Bestimmung des E-Moduls in einer schematischen Darstellung;

Fig.18 die Einspannvorrichtung zur Bestimmung der Torsionsfrequenz in einer schematischen Darstellung;

Fig.19 die Vorausberechnung der 28 Tage Druckfestigkeit.

Im Rahmen des erfindungsgemäßen automatisierten Prüfstandes steht die eindeutige und zuverlässige Kennzeichnung und Identifikation der Probekörper an erster Stelle. Im Folgenden werden die Anforderungen an die Probenkennzeichnung und die Möglichkeiten zur Kennzeichnung und Identifikation der Prismen beschrieben.

An die Kennzeichnung der Prüfkörper werden eine Reihe von Anforderungen gestellt. Damit bei einer späteren Ausweitung des Anwendungsbereiches keine Probleme entstehen, werden auch andere Prüfverfahren und die daraus entstehenden Anforderungen berücksichtigt. Hierzu zählen die Besonderheiten des Angriffs durch saure und sulfathaltige Wässer. Es ergibt sich nachstehender Anforderungskatalog an das Kennzeichnungssystem:

- Kennzeichnung auf oberflächenfeuchten Mörtelprismen

- Kein Einfluss durch Wasserlagerung bei 20°C

- Auswertbare Identifikation bei Lagerung in sulfathaltigen Wässern mit einer Konzentration von max. 24.000 mg/l $SO_4^{2-}$

- Auswertbare Identifikation bei Lagerung in sauren Wässern mit pH-Wert >4,0

- Beständigkeit der Probenidentifikation bei gegebenen Lagerungsbedingungen

- Auswertbare Identifikation bei Schwinden oder Treiben

- Mörteloberflächeneigenschaften (Unebenheiten durch Poren, Bluten, Absanden) dürfen keinen Einfluss nehmen

- Anbringung muss spannungsfrei erfolgen

- Kennzeichnung darf keinen Einfluss auf den zu ermittelnden Festigkeitsverlauf haben

- Kennzeichnung sollte keinen oder gleichbleibenden Einfluss auf die Prüfgrößen haben

Auf der Basis des Anforderungskataloges sind verschiedene erfindungsgemäße Möglichkeiten der Kennzeichnung verwendbar.

Die Kennzeichnung der Probekörper ist wesentlicher Bestandteil der Erfindung, da ohne eine eindeutige und dauerhafte Probenkennzeichnung eine bedienerfreie Automatisierung nicht realisierbar ist. Für den Fall eines Ausfalls des Prüfstandes muss die Identifikation der Probekörper auch ohne erhöhten Aufwand möglich sein. Aus diesem Grund sollte die Kennzeichnung über mindestens zwei verschiedene Möglichkeiten erfolgen.

Eine erste erfindungsgemäße Ausführungsform sieht vor, die Prüfkörper durch Bedrucken mit Informationen zu versehen.

Aufgrund der bestehenden Anforderungen muss die Tinte, mit der die Informationen aufgesprüht werden, wasserbeständig sein. Es wurden Prismen im oberflächenfeuchten Zustand mit einer wasserbeständigen Tinte besprüht. Aus Kontrastgründen wurden für diesen Probedruck ein Prisma aus einem Grauzement und ein Prisma aus einem Weißzement mit der längsten handelsüblichen Zementbezeichnung, dem Herstelldatum, der Labornummer und einem der Labornummer entsprechenden Barcode bedruckt. Nach dem Bedrucken wurden die Prismen 14 Tage unter Wasser bei den nach EN 196-1 genormten Bedingungen gelagert.

Beim Vergleich der Prismen vor und nach der Lagerung können keinerlei wesentliche Unterschiede festgestellt werden. Somit ist dieses Verfahren zur Kennzeichnung der Prismen sehr gut geeignet. Diese Kennzeichnungsvariante kann jedoch nur für die Lagerung in Wasser oder sulfathaltigen Wässern verwendet werden, da bei einem Angriff saurer Wässer die Mörteloberfläche und dadurch auch die Kennzeichnung beschädigt wird.

Die Identifikation des Barcodes kann maschinell über einen Scanner erfolgen. Zusätzlich kann im Falle eines Ausfalls des Prüfstandes die Identifikation durch einen Laboranten vorgenommen werden.

Vorteile dieser Kennzeichnungsvariante:

- Dauerhaftigkeit bei Wasserlagerung

- Einfache maschinelle Identifikationsmöglichkeit über Laserscanner

- Im Notfall Möglichkeit der schnellen Identifikation über die aufgedruckte Labornummer

- Keinerlei Einflussnahme der Kennzeichnung auf Erhärtungsverlauf

- Keinerlei Einflussnahme der Kennzeichnung auf weitere Messverfahren

Eine weitere erfindungsgemäße Ausführungsform sieht vor, bedruckte Etiketten auf den Prüfkörpern zu befestigen. Hierfür werden zunächst weiße wasserbeständige Etiketten bedruckt. Diese werden dann auf die Prismen geklebt. Bei den Etikettendruckspende-Systemen gibt es zwei unterschiedliche Technologien:

- Thermodirekt
- Thermotransfer

Bei der Thermodirekt-Technologie werden die Informationen auf ein thermisch empfindliches Etikettenmaterial eingebrannt. Je nach Ausführung des Systems kann die Auflösung bis zu 600 Punkte pro Zoll betragen. Bei der Thermotransfer-Technologie erfolgt die Beschriftung des Etiketts über eine Folie. Dabei wird das Schriftbild auf die Etiketten aufgeschmolzen. Der Vorteil dieser Technik ist, dass das Schriftbild haltbarer und besser ist. Durch Verwendung farbiger Folien können sogar farbige Etiketten hergestellt werden.

Die Möglichkeit durch weiße oder farbige Folien bietet zusätzliche Gestaltungsvarianten hinsichtlich der Farbkennzeichnung verschiedener Zemente.

Vorteile gegenüber dem Ink Jet Verfahren

- Geringere Anschaffungskosten der Geräteausstattung

- Etiketten können in trockenen Zustand im Voraus bedruckt werden

Ferner kann erfindungsgemäß ein Radiofrequenz-Identifikationssystem (RFID) eingesetzt werden.

Diese Radiofrequenz-Identifikationssysteme (RFID-Systeme) ermöglichen eine berührungslose Probekörperidentifikation. Diese batterielosen Datenträger arbeiten mit einem eingebrannten, unveränderbaren Festcode von maximal 96 Bit. Je nach Ausführung der Datenträger können diese auch mit wichtigen Produktionsdaten beschrieben und später bei der Identifikation ausgelesen werden.

Die RFID-Systeme werden in verschiedenen Varianten angeboten:

- Schreib- / Lesesysteme mit passiven Datenträgern

- Schreib- / Lesesysteme mit aktiven Datenträgern

- Lesesysteme mit passiven Datenträgern

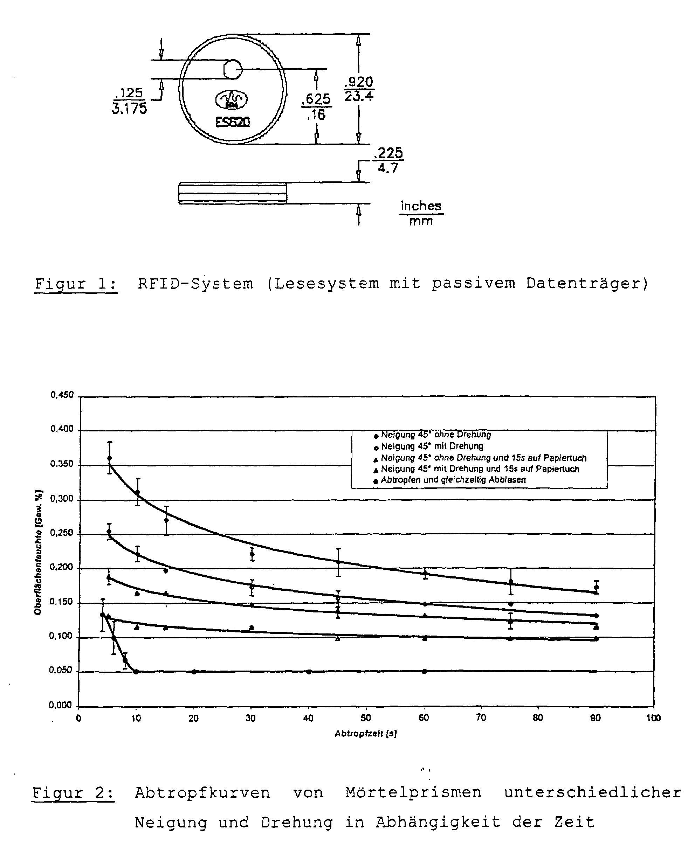

Für die Umsetzung der Prüfstandsapparatur steht lediglich die Erkennung der Probekörper im Vordergrund. Aus diesem Grund wurden die Lesesysteme mit passiven Datenträgern für einen späteren Einsatz ausgewählt (siehe Figur 1).

Die Speicherung der im Prüfzeitraum ermittelten Daten soll über einen externen Computer erfolgen. Die Einsatztemperaturen liegen je nach Ausführung zwischen -20°C und 85°C. Die Codierung hat eine Länge von 20 Bit, wodurch eine Million mögliche Codierungen realisiert werden können. Die Leseköpfe zum Auslesen der Bauteilkennung haben einen Durchmesser von 18 mm und unterscheiden sich nach der horizontalen oder vertikalen Ausrichtung des Lesekopfes. Diese besitzen jeweils eine integrierte Antenne mit einer Reichweite zum Auslesen von maximal 66 mm. Aufgrund des geringen Leseabstandes ist die Antenne direkt am Greifarm des Roboters zur Ein- und Auslagerung der Prismen anzubringen. Die Datenträgerkennung wird über eine RS 232 Schnittstelle eines angeschlossenen Computers und dem Hyper Terminal Programm unter dem Betriebssystem Windows ausgelesen und im ASCII-Format abgespeichert. Für den Betrieb der Antenne ist eine Spannungsversorgung mit 24 V erforderlich.

RFID-Systeme bieten gegenüber Barcode-Systemen oder anderen optischen Erkennungssystemen zahlreiche Vorteile:

- Unempfindlich gegenüber Einwirkung von Feuchtigkeit, Kälte, Hitze, Schmutz, Staub

- Unempfindlich gegenüber Einwirkung von Stößen Vibrationen und elektromagnetischen Störfeldern

- Unempfindlich gegenüber sauren Umgebungsmedien

- Robuste wiederverwendbare Datenträger

- Keine Beeinträchtigung der Datenübertragung durch nichtleitende Materialien

- Keine Sichtverbindung zu den zu identifizierenden Objekten erforderlich

- Hohe Zuverlässigkeit der Datenübertragung (Fehlerquote 1 zu 100 Trillionen)

- Keine zusätzlichen Systeme zur Aufbringung nötig

- Identifikation auch unter Wasser möglich

Die hohe Zuverlässigkeit in der Datenübertragung ist in direktem Zusammenhang mit dem Verbund zwischen dem Tag und dem Prüfkörper zu sehen. Die Ankopplung des Bauteils muss für den Prüfzeitraum stark genug sein, aber nach Ablauf der Prüfung sich auch leicht lösen lassen, um die Wiederverwendbarkeit der Bauteile zu sichern.

Als besonders geeignetes Verbindungsmedium hat sich Butyl-Dichtungsband erwiesen.

Das Allzweck-Dichtungsband auf Butylbasis sorgte über den gesamten Verlauf der Wasserlagerung für einen sicheren Verbund. Zur weiteren Verbesserung der Haftfestigkeit und leichteren Anbringung des Datenträgers kann das Butylband nach dessen Aufbringung kurz erwärmt werden, bevor der Datenträger aufgedrückt wird.

Trotz der hohen Zuverlässigkeit dieses Systems kann es vorteilhaft sein, es mit einem zweiten System zu kombinieren. Zusätzliche Sicherheit oder eine Erhöhung der Prozessgeschwindigkeit kann über die Lage der Prismen im Wasserbecken oder auch über das Ink Jet Verfahren gewonnen werden.

Die günstigste Möglichkeit ist die Identifikation über die Lage der Prismen im Wasserbecken. Hierzu werden die Wasserbecken in einzelne Lagerplätze aufgeteilt und mit xy-Koordinaten versehen. Damit der Roboter beim Einlagern und Auslagern fehlerfrei und problemlos arbeiten kann, sollten Kunststoffprofile in die Becken und an die Wartepositionen, wie Messstellen und Einlagerungszone, angebracht werden. Diese Profile sorgen für eine genaue Positionierung der Probekörper und erleichtern das Greifen des Roboters.

Ein derartiges Kunststoffprofil kann beispielsweise ein flacher Gitterkorb aus Kunststoff sein, welcher von oben beschickbare einzelne Fächer aufweist, welche durch Gitterwandungen voneinander abgeteilt sind. Die Grundfläche dieser Abteile ist dabei deutlich größer als die Grundfläche eines Prüfkörpers, wobei an den Abteilungswänden nach oben weisende, beispielsweise pfeilförmige Führungseinrichtungen vorhanden sind, welche über nach unten und innen verlaufende Schrägflächen verfügen. Wird ein Prüfkörper durch den Roboterarm nicht genau an dem gewünschten Ort im Abteilungsfach abgesetzt kann der Prüfkörper somit an diesen Schrägflächen entlang und in sein Abteil hinein rutschen. Hierdurch wird eine koordinatengenaue Ablegung des Prüfkörpers im Kunststoffkorb auch dann erreicht, wenn der Roboterarm aus irgendeinem Grund keine exaktkoordinatengenaue Ablage bewerkstelligt. Die Vorrichtungen zum Positionieren des Prüfkörpers, beispielsweise die pfeilartigen Elemente, sind dabei bezüglich ihrer Breite so gewählt, daß der Prüfkörper nur teilbereichsweise auf ihnen aufliegt, so daß eine gleichmäßige Umströmung aller Prüfkörper mit Wasser jederzeit gewährleistet ist.

Um eine hohe Zuverlässigkeit zu erreichen, müssen hohe Anforderungen an die Software zur Lagerplatzverwaltung gestellt werden. Um Verwechslungen von Probekörpern oder Vertauschen von Lagerplätzen auszuschließen, müssen die Lagerplätze für die Dauer des Messzeitraumes, in der Regel 28 bzw. 56 Tage, einer Probenummer zugeordnet werden können. Erst nach Ablauf der Messung darf der entsprechende Lagerort freigegeben werden.

Eine weitere Ausführungsform sieht die Lagerung der Prismen in einem klimatisierten Aktenschrank vor, der nach dem Paternosterverfahren arbeitet. Hierbei werden die Prismensätze jeweils einzeln in Kunststoffwannen mit eingeprägten Profilen gelagert. Auf diese Art und Weise ist die nach Zementsorten getrennte Lagerung sehr leicht zu realisieren. Ein weiterer Vorteil dieser Lagerungsvariante ist die Ausnutzung der Raumhöhe. Aufgrund der Ausnutzung der Raumhöhe kann die Linearachse bei der Auslegung des Robotersystems verkürzt werden.

Auch diese Variante wird vorzugsweise in Verbindung mit einer anderen genutzt, wobei das RFID-System hierfür das geeignetste ist, da es auch eine Identifikation unter Wasser ermöglicht.

Eine weitere Ausführungsform sieht lasergeprägte Kunststoffplatten zur Identifizierung vor.

Beim Laserprägen wird auf eine dünne Kunststoffplatte oder auch -folie mit einem Laser ein Barcode oder auch andere Informationen eingebrannt. Zur Kennzeichnung muss diese Platte wiederum fest mit dem Prüfkörper verbunden werden. Da diese Kunststoffplatten nicht wiederzuverwenden sind, muss die Ankopplung nicht wieder zu lösen sein. Je nach Größe der Kunststoffplatte und dem Ort ihrer Anbringung kommt es zu einer Einflussnahme auf die Ergebnisse der Längen- bzw. der Resonanzfrequenzmessung. Beides ist bei gleichbleibender Größe, Materialbeschaffenheit und Ort der Anbringung konstant und lässt sich somit berücksichtigen. Die Kunststoffplättchen sind bei der Herstellung des Mörtels anzubringen.

Ferner ist auch ein Nadelprägen der Mörteloberfläche möglich.

Beim Nadelprägen wird durch Einstanzen von Ziffern und Buchstaben in die Mörteloberflächen der Prüfkörper gekennzeichnet. Diese Art der Kennzeichnung muss im Gegensatz zu den bis hierher vorgestellten Varianten direkt im Anschluss an die Herstellung der Probekörper und nicht an den bereits erhärteten Probekörpern erfolgen.

Da der Kennzeichnung und Identifikation der Prüfkörper ein sehr hoher Stellenwert zuzuordnen ist, ist es vorteilhaft, mindestens zwei Kennzeichnungssysteme zu verwenden. Jede Kombination soll bei einem Ausfall eine eindeutige Identifikation sicherstellen.

Folgende Kombinationen sind vorteilhaft ausführbar:

- RFID / Ink Jet / Lage im Wasserbecken

- RFID / Etiketten / Lage im Wasserbecken

- RFID / Lage im Wasserbecken

Die ersten beiden Varianten bieten über die RFID-Systeme eine hohe Zuverlässigkeit und die Möglichkeit der Identifikation unter Wasser. Weiterhin kann eine zusätzliche Kontrolle durch einem Laboranten bei einem Ausfall des Systems über den aufgebrachten Barcode erfolgen. Durch die Kombination von drei Identifikationssystemen erhöht sich die Sicherheit, sowohl für den Betrieb als auch bei Ausfall von Teilen des Identifikationssystems. Durch die Verwendung von drei Kennzeichnungssystemen steigen auch die Kosten. Vor allem die Kennzeichnung mit dem Ink Jet Verfahren lässt die Kosten stark ansteigen.

Die letztgenannte Variante ist die kostengünstigste Möglichkeit einer zuverlässigen Kennzeichnung und Identifikation. Hierbei entfällt jedoch die einfache Identifikation über den Barcode. Da für das Laborpersonal ohne Hilfsmittel keinerlei Möglichkeit besteht, einen einzelnen Probekörper zu identifizieren, muss die Zuverlässigkeit an den Prüfstand sehr hoch sein. Bei dieser Kennzeichnungsvariante kann die Identifikation außerhalb des Lagerplatzes nur mit Hilfe einer Antenne erfolgen.

Nach der Kennzeichnung der Prüfkörper erfolgt eine Volumenund Massebestimmung derselben.

Die Bestimmung der Masse und des Volumens erfolgt im wesentlichen aus zwei Gründen. Der Quotient aus der bestimmten Masse und dem Volumen geht als Prüfrohdichte in die Berechnung des dynamischen Elastizitätsmoduls ein. Desweiteren können durch die Volumen- und Masseänderung die Erscheinungen wie Schwinden, Quellen und Treiberscheinungen verfolgt werden.

Die Massebestimmung der Prismen zur Ermittlung der Prüfrohdichte erfolgt an oberflächenfeuchten Prismen. Hierfür bieten sich verschiedene Möglichkeiten an, eine vergleichbare Oberflächenfeuchte zu erzielen.

- Abtropfen der Prismen

- Abtropfen in Verbindung mit der Ablage auf einer saugenden Unterfläche

- Abblasen der Mörtelprismen mittels Druckluft

Um die Resonanzfrequenzmessung unter dem Einfluss einer konstanten Oberflächenfeuchte durchführen zu können, werden Abtropfkurven in Abhängigkeit zur Zeit bestimmt. Innerhalb eines Abtropfversuches werden drei Mörtelprismen aus der Wasserlagerung herausgehoben, die in bestimmten Neigungen unterschiedlich lange abtropften. Durch Wägung der oberflächenfeuchten und oberflächentrockenen Prismen kann der an der Oberfläche verbleibende Wassergehalt bestimmt werden. In Figur 2 sind die Kurvenverläufe verschiedener Neigungen und Drehungen um die Längsachse über der Abtropfzeit dargestellt. In einem weiteren Versuch wurden die Prismen nach der jeweiligen Abtropfzeit auf ein wassersaugendes Papiertuch gesetzt. In einem vierten Versuch tropften die Prismen unter einer Neigung und Drehung von 45° ab und wurden gleichzeitig mit einem Druckluftstrahl von 2 bar abgeblasen.

Anhand der Figur 2 ist zu erkennen, dass durch reines Abtropfen die verbleibende Oberflächenfeuchte bei etwa 0,1 Gew. % liegt. Die verbleibende Oberflächenfeuchte kann durch zusätzliches Abblasen mit Druckluft um weitere 50 % reduziert werden. Weiterhin kann die Dauer bis zum Erreichen der Gewichtskonstanz mit 10 s auf etwa 1/3 reduziert werden.

In der nachstehenden Tabelle 1 sind die Zeiten bis zur Gewichtskonstanz und die verbleibende Oberflächenfeuchte gegenübergestellt.

Tabelle 1:

| Vergleich verschiedener Verfahren zur Einstellung konstanter Oberflächenfeuchte nach der Wasserlagerung der Mörtelprismen | | |
|---|---|---|
| Abtropf-Verfahren | Zeit bis zur Gewichtskonstanz [s] | Verbleibende Oberflächenfeuchte [Gew.-%] |
| 45°Neigung und ohne Drehung | 90 | 0,17 |
| 45°Neigung und 45° Drehung und Längsachse | 90 | 0,13 |
| 45°Neigung und 45° Drehung und Längsachse und saugende Unterlage | 45 | 0,10 |
| 45°Neigung und 45° Drehung und Längsachse gleichzeitiges Abblasen | 10 | 0,05 |

Für das Erreichen einer vergleichbaren Oberflächenfeuchte mittels Abtropfen wird erfindungsgemäß anhand der erstellten Kurven eine Abtropfzeit von 45 Sekunden bei einer Neigung von 45° und einer Drehung um 45° um die Längsachse verbunden mit anschließender Auflage auf eine wassersaugende Unterlage vorgeschlagen.

Um die Arbeitsgeschwindigkeit beim Auslagern der Prismen zu erhöhen, ist ein schnelles Erreichen der Gewichtskonstanz erforderlich. Aus diesem Grund werden die Prismen mit einer Neigung von 45° zur Wasseroberfläche und einer Drehung um 45° um die Längsachse aus dem Wasserbecken entnommen und gleichzeitig für 10 Sekunden mit einem Druckluftstrahl von etwa 2 bar abgeblasen. Mit Hilfe dieses Verfahrens kann die in Form eines dünnen Films an der Oberfläche der Prismen verbleibende Oberflächenfeuchte auf 0,05 Gew. % reduziert werden. Die Massebestimmung erfolgt somit mit einem konstanten Fehler aufgrund des anhaftenden Wassers.

Die Messgenauigkeit der Wägeinrichtung beträgt 0,1 g. Das Ergebnis der Massebestimmung wird über eine RS 232 Schnittstelle an einen Prozessrechner übermittelt und dem entsprechenden Datensatz zugeordnet.

Für die Berechnung der Prüfrohdichte, die ihrerseits in die Berechnung des dynamischen Elastizitätsmodul $E_D$ und des Schubmoduls $G_T$ eingeht, muss eine Volumenbestimmung erfolgen. Die Genauigkeit des Messsystems ergibt sich aufgrund des Anwendungsbereiches der Prüfstandsapparatur. An dieser Stelle sollten zwei verschiedene Anwendungsbereiche unterschieden werden. Zur reinen Bestimmung der Druckfestigkeit nach DIN EN 196-1, bei der die Charakterisierung der Volumenänderung nicht im Vordergrund steht ist eine Messgenauigkeit von ± 0,1 mm zur Volumenbestimmung und späteren Berechnung der Prüfrohdichte ausreichend. Bei einer zusätzlichen Anwendung im Hinblick auf die Charakterisierung und Analyse von Schadreaktionen, die in Verbindung mit einer Volumenänderung stehen, ist die Messgenauigkeit von ± 0,1 mm nicht mehr ausreichend. Für den Nachweis von Treiberscheinung gilt der Grenzwert von 0,1 mm/m hinsichtlich Längenänderung, umgerechnet auf die Länge der Mörtelprismen entspricht dieser Grenzwert einer Längenänderung von 0,16 mm. Aus diesen Überlegungen heraus ergibt sich eine Messgenauigkeit von mindestens ± 0,01 mm. Zur Erhöhung der Aussagekraft und zur frühen Erkennung von Treiberscheinungen soll die Längenmessung nicht nur eindimensional, sondern dreidimensional erfolgen.

Für die Bestimmung des Volumens lassen sich nachfolgende Verfahren verwenden:

• 3-D-Vermessung mittels Lasertriangulation

• 3-D-Vermessung mittels induktiver Wegaufnehmer

Die einzelnen Möglichkeiten werden im Folgenden näher erläutert.

Eine erste Möglichkeit ist die Lasertriangulation. Ein Laserstrahl wird auf ein Werkstück gerichtet. Am Auftreffort wird das Licht gestreut. Das Streulicht wird für die Beobachtung des Auftreff-ortes ausgenutzt. Bei einer ideal spiegelnden Oberfläche tritt kein diffuses Streulicht auf, sondern nur eine gerichtete Reflexion. In diesem Fall eignet sich dieses Verfahren nicht zur Vermessung. Im Allgemeinen hängt das Streuverhalten von den Materialeigenschaften, seiner Oberflächenstruktur und der Wellenlänge des Lasers ab. Als Detektoren für die Abbildung des Streulichtes dienen Lateraleffektdioden oder CCD-Zeilen. Aus der gemessenen Lage des Bildfleckes sowie der bekannten Abbildungsgeometrie und Einstrahlrichtung wird der Abstand des Messobjektes in z-Richtung von einer Referenzebene (xy-Ebene) bestimmt. Bei diesem Verfahren werden hauptsächlich HeNe-Laser und Halbleiterlaser eingesetzt. Letztere werden kontinuierlich oder gepulst betrieben. Die Strahlleistung der Halbleiterlaser lässt sich elektronisch steuern. Damit ist es möglich, starke Schwankungen, die durch unterschiedliche Oberflächeneigenschaften hervorgerufen werden, auszugleichen.

Die Prüfgenauigkeit bei der Verwendung und Auswertung des Leuchtfleckes beträgt 10 µm. Zur Vermessung der gesamten Prismenoberfläche müssen die Laser-Triangulationssensoren an einen Koordinatenmesstisch angebracht werden, mit dessen Hilfe die Oberfläche in frei wählbaren Abständen abgerastert werden kann. Mit Hilfe dieses Verfahrens lässt sich zusätzlich auch eine mögliche Wölbung der Prismen durch Treiberscheinungen verfolgen.

Für die Wegmessung über induktive Wegaufnahme werden in der Regel passive induktive Aufnehmer verwendet. Hierbei verändert ein axial im Aufnehmer verschiebbarer ferromagnetischer Kern die Induktivität der Messspule und verursacht damit eine dem Weg proportionale Aufnehmerausgangsspannung. Der verschiebbare Tauchanker ist mit Teflon beschichtet, um die Reibung zwischen Kern und Kernkanal zu verringern. Das Auflösungsvermögen ist dabei nahezu unendlich groß, da es nur durch die Rauschspannung des Aufnehmers und der Elektronik begrenzt wird. Die kleinste noch messbare Bewegung liegt bei $10^{-5}$ mm. Allerdings treten bei solchen Messungen Probleme durch kleinste Temperaturschwankungen (durch Wärmedehnung der Werkstoffe) auf.

Die im Rahmen der Erfindung verwendeten induktiven Wegaufnehmer arbeiteten mit einer Messgenauigkeit von 0,001 mm (1 µm $\cong$ kleines Zementkorn).

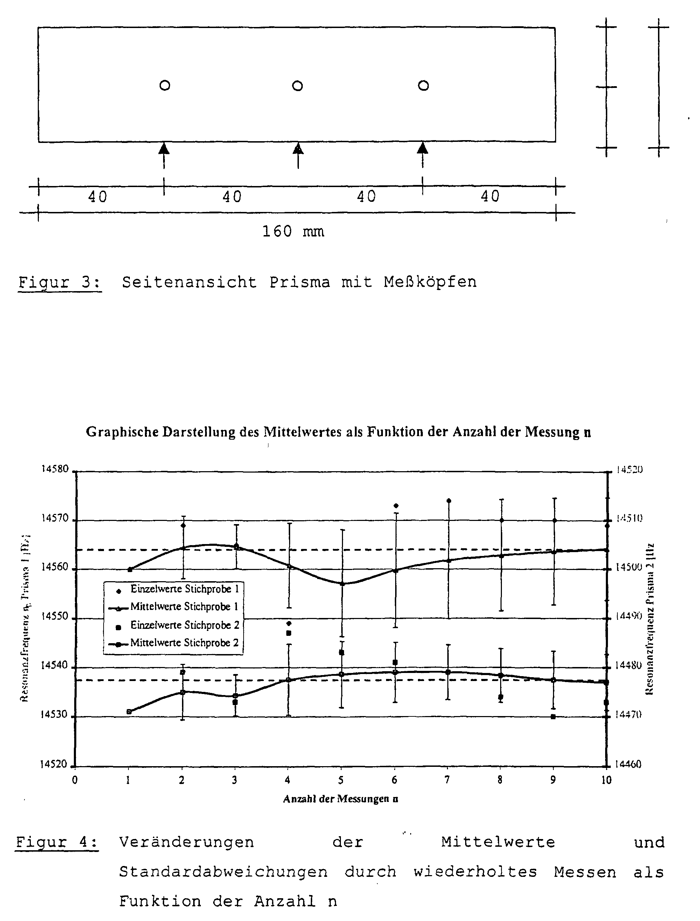

Aufgrund der schlanken Form der Mörtelprismen, deren Verhältnis von Länge zu Breite und Höhe 4:1:1 beträgt, empfiehlt es sich, die Höhe und Breite der Prismen an mindestens drei Stellen zu messen und den Mittelwert für die weiteren Berechnungen zu verwenden. Eine mögliche Anordnung der Messköpfe ist in Figur 3 dargestellt. Die Seitenansicht und die Draufsicht sind identisch nur um 90° gedreht.

Im Unterschied zur Unterwasserwägung lassen sich aufgrund der Einzelmessungen sowohl die Dehnungen in den jeweiligen Raumrichtungen als auch die Volumendehnung berechnen. Im Hinblick auf die Graf-Kaufmann-Methode kann aufgrund der ermittelten Volumendehnung von einer höheren Aussagekraft ausgegangen werden.

Vorteile gegenüber der Messung mit einem Laser:

• Geringer apparativer Aufwand

• Geringer Anschaffungspreis trotz hoher Genauigkeit

• Geringe Betriebskosten

• Kein Einfluss der Oberflächenfeuchte auf das Messergebnis

Die Vermessung mit Hilfe eines Lasersystems ist für beide anfangs genannten Anwendungsgebiete einsetzbar. Für die geringe Anforderung hinsichtlich der Volumenbestimmung bei der angestrebten Normüberwachung ist der Einsatz der induktiven Wegaufnehmer ausreichend.

Es gibt mehrere grundsätzliche Möglichkeiten, anhand von Ultraschallwellen Aussagen über Werkstoffeigenschaften zu gewinnen. Je nach Art und Weise, wie der Schall im Werkstoff erzeugt bzw. auf den Werkstoff übertragen und von diesem wieder empfangen wird, unterschiedet man folgende Anwendungen:

• Schallemissionsanalyse (acoustic emission analysis)

• Durchschallungsmethode (transmission method)

• Impuls-Echo-Methode (puls-echo method)

• Resonanzfrequenzmethode (resonant frequency method)

Nachfolgend werden die genannten Verfahren genauer beschrieben.

Bei der Schallemissionsanalyse werden die durch innere Spannungen oder bei Belastungen eines Bauteils abgegebenen (emittierten) Schallwellen an dessen Oberfläche empfangen, ausgewertet und gegebenenfalls auch der Entstehungsort geortet. Grundlegender Effekt hierbei ist, dass ein wachsender Riss Energie freisetzt, die sich in Form von Ultraschallwellen fortsetzt.

Für Beton findet dieses Verfahren die häufigste Anwendung. Hiermit sind in der Vergangenheit fast alle Versuche durchgeführt worden. Schallsender und -empfänger sind an genau gegenüberliegenden Seiten des Bauteils oder Probekörpers der Dicke 1 angebracht.

Hierbei ist von Nachteil, dass das Bauteil von beiden Seiten zugänglich sein muss.

Zur Auswertung des empfangenen Ultraschallsignals wurde in der Vergangenheit in der Regel nur die Messung der Laufzeit t, die der Impuls für die Bauteildicke 1 benötigt, herangezogen. Die meisten Geräte messen nur die Laufzeit, nur wenige messen auf einem zweiten Kanal die Schalldruckamplitude. Seit einigen Jahren werden Ultraschallgeräte gebaut, die zusätzlich noch eine Frequenzanalyse durchführen können. Dabei wird der empfangene Schallimpuls, der einen Frequenzumfang von 300 kHz besitzt, in mehrere Frequenzbereiche (z.B. 15 Bereiche mit 20 kHz) zerlegt. Von diesen Teilbereichen wird dann die Intensität des Schallimpulses registriert. Zu der Intensität des Gesamtimpulses erhält man noch so viele Intensitätswerte, wie Frequenzbereiche vorhanden sind.

Während die Messungen am luftgelagerten Festbeton gute Übereinstimmungen zeigen, weichen die Messungen an den wassergelagerten Mörtelprismen stark von den zerstörend gemessenen Druckfestigkeiten ab. Die im Programm eingebrachten Formeln gehen von luftgefüllten Poren aus. Die Schallgeschwindigkeit ist in Wasser sehr viel höher als in Luft, wodurch die für den jeweiligen Prüfkörper gemessene Schallgeschwindigkeit zu hoch war. Die berechneten Druckfestigkeiten wurden aufgrund der hohen Schallgeschwindigkeit zu hoch errechnet und lagen deutlich über den zerstörend ermittelten Druckfestigkeiten.

Berührungsfrei arbeitende Prüfverfahren auf der Grundlage der Durchschallungsmethode werden im Rahmen des CIF-Tests zur Charakterisierung der Schädigung nach Frost-Tau-Wechseln verwendet. Hierbei dient die Veränderung des dynamischen Elastizitätsmoduls als Maß für die Schädigung.

Eine weitere Möglichkeit ist das Impuls-Echo-Verfahren.

Die Impuls-Echo-Methode bietet den großen Vorteil gegenüber den anderen genannten Verfahren, Messungen auch an Bauteilen vornehmen zu können, die nur von einer Seite zugänglich sind. Hierbei sind Schallsender und -empfänger in einem Prüfkopf vereint. Gemessen werden die Schallreflexionen aufgrund von Sprüngen im Schallwiderstand, die wiederum durch Fehlstellen oder Probekörperoberflächen entstehen.

Bei der Untersuchung metallischer Werkstoffe ist dieses Verfahren hochentwickelt, bei Betonen liegen allerdings erst orientierende Untersuchungen vor. Dabei hat sich für Beton der Frequenzbereich von 20 - 300 kHz als günstig erwiesen. Eine höhere Frequenz bewirkt einen starken Anstieg der Schallschwächung. Verwendet man für Beton den Frequenzbereich von Metallen, erhält man Wellenlängen, die den Durchmesser des Sandes (bis 4 mm) entsprechen. Die Folge ist ein starker Anstieg der Streuung, d.h. eine hohe Schallschwächung. Infolge der niederfrequenten Prüfköpfe besteht zudem keine Richtcharakteristik. Die daher überall im Beton stattfindenden Reflexionen gelangen aufgrund der kugelförmigen Schallausbreitung auch aus allen Richtungen wieder zum Prüfkopf zurück und bewirken Störechos.

Diese Schwierigkeiten, insbesondere die Unterscheidung der relevanten Reflexionen von den Störechos, haben dazu geführt, dass sich die Impuls-Echo-Methode noch nicht durchgesetzt hat.

Erfindungsgemäß bevorzugt wird die Resonanzfrequenzmethode.

Bei der Resonanzfrequenzmethode wird der zu untersuchende Probekörper mit Ultraschall, der in der Frequenz stufenlos einstellbar ist, angeregt. Damit lässt sich seine Eigenschwingung und somit seine Eigenfrequenz $f_r$ (Resonanzfrequenz) ermitteln. Die Eigenfrequenz eines Probekörpers hängt von seinen Abmessungen und seinen elastischen Eigenschaften (den Materialkonstanten E und p) ab. Im Versuch werden aus den gemessenen Eigenfrequenzen und den bekannten Abmessungen die Materialkonstanten bestimmt. Je nach Art der Anregung erhält man bevorzugt Längs-, Torsions- oder Biegeschwingungen in Form von stehenden Wellen mit ausgeprägter Amplitude.

Bei der Dehnwelle oder Longitudinalwelle treten die Schwingungsbäuche an beiden Stirnseiten auf, wo auch Geber und Aufnehmer, möglichst in der Mitte, angekoppelt werden. Die Prismen werden zur einwandfreien Bestimmung der Eigenfrequenz auf eine mittig angeordnete stark dämpfende Unterlage (Schaumstoff) gelegt.

Die Torsionswelle oder Transversalwelle hat ihre Schwingungsbäuche ebenfalls an den Prismenenden. Die Auflagepunkte von Geber und Aufnehmer befinden sich diagonal an einer Seitenfläche. Analog der Bestimmung der Dehnwelle wird die dämpfende Unterlage mittig angeordnet.

Bei der Biegewelle liegen die Schwingungsbäuche an den Enden und in der Mitte des Prüflings. Die Schwingungsknoten befinden sich im Abstand von 0,224 % oder 3,58 cm der Prüfkörperlänge von den beiden Stabenden entfernt. Geber und Aufnehmer liegen an den Enden des Prüflings mittig auf.

Bei gleichbleibenden Probekörperdimensionen stehen die drei Grundresonanzen in einem bestimmten Verhältnis zueinander. Für die hier gemessenen Mörtelprismen ist das Verhältnis der Dehn, Torsions- und Biegewelle 1: 0,58:0,43. Stimmt dieses Verhältnis, dann kann man davon ausgehen, dass die Messung der Grundresonanzen korrekt vorgenommen worden ist.

Für eine korrekte und vergleichbare Messung der Grundresonanzen müssen eine Reihe von Faktoren und Anforderungen berücksichtigt werden. Dazu gehören:

- Geräteschwankung

- Ankopplungsdruck

- Einfluss der Oberflächenfeuchte

- Mindestprobenalter

Zur Bestimmung der Geräteschwankungen werden zwei Prüfkörper zehnmal gemessen. Die Prismen haben ein Alter von mehr als 90 Tagen. Ein Prisma ist mit einem Portlandzement das andere ist mit einem geschlackten Zement hergestellt worden. Zwischen den einzelnen Messungen wurden die Prüfköpfe immer wieder abgekoppelt und die Resonanzfrequenz erneut gemessen.

Anhand der errechneten Wiederholstreuung dieser Wiederholungsprüfungen zeigt sich die Güte des Verfahrens. Der Güte hinsichtlich der Schwankung der gemessenen Grundfrequenzen befindet sich unter 0,1 %. Aufgrund dieser geringen Schwankungen eignet sich dieses Verfahren sehr gut für die weiteren Versuche. Übertragen auf die Berechnung der Druckfestigkeiten zieht eine fehlerhafte Messung von 10 Hz eine Abweichung von 0,5 % und bei 20 Hz von 1,0 % nach sich.

Anhand der ermittelten Werte sind für Figur 4 die Mittelwerte und Standardabweichungen nach jeder weiteren Messungen berechnet worden. Aufgrund der ermittelten Werte soll untersucht werden, ob sich die Mittelwerte und Standardabweichungen durch wiederholtes Messen verändern.

Anhand von Figur 4 zeigt sich, dass der durch nur eine Messung ermittelte Wert mit einen Fehler von etwa 0,04 % den Mittelwert aus 10 Messungen wiederspiegelt. Mit zwei Messungen wird der Mittelwert bereits exakt abgebildet. Die Schlussfolgerung aus dieser Untersuchung ist, dass das Resonanzfrequenzverfahren mit äußert geringen Schwankungen arbeitet. Übertragen auf die Bestimmung des dynamischen Elastizitätsmoduls $E_D$ bedeutet dies, dass der Mittelwert von drei gemessenen Prismen als korrektes Ergebnis angesehen werden kann.

Es wurde herausgefunden, dass ein qualitativer Einfluss des Anpressdruckes der Messköpfe besteht. Bei der Durchschallungsmethode mit Prüfköpfen von 22,6 mm Durchmesser konnte ermittelt werden, dass der Energieverlust, ausgedrückt durch die Verstärkung V des Messsignals zwischen Prüfkopf und Probekörper, ab einer Anpresskraft von 35 N konstant bleibt. Diese Kraft entspricht einem Anpressdruck von 0,09 N/mm$^2$.

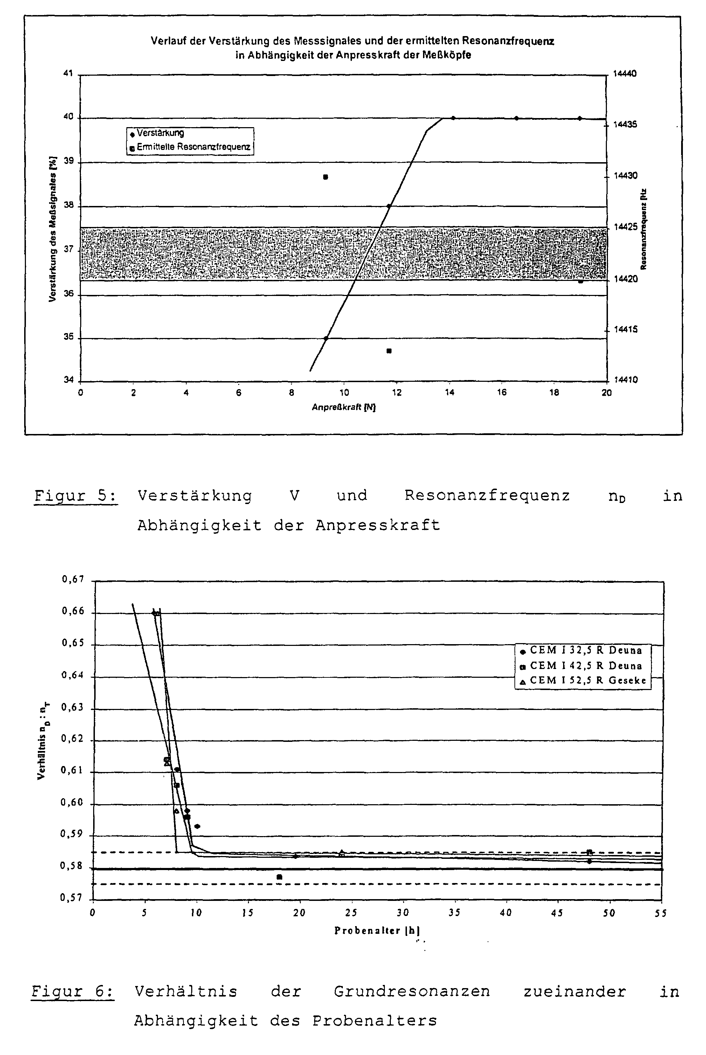

Die Tastspitzen der Prüfköpfe bei der Resonanzfrequenzbestimmung haben einen Durchmesser von 2 mm. Figur 5 zeigt die Verstärkung des Messsignals sowie die ermittelte Resonanzfrequenz in Abhängigkeit der aufgebrachten Kraft.

Anhand von Figur 5 zeigt sich, dass ab einer Anpresskraft von 14 N die Verstärkung nicht weiter zunimmt. Die Anpresskraft von etwa 9 N entspricht dem Eigengewicht des Prüfkopfes. Die Anpresskraft von 14 N entspricht einem Anpressdruck von ca. 1,1 N/mm$^2$. Da im Gegensatz zur Durchschallungsmethode keine Ankopplungshilfe in Form von Silicongel verwendet wird, muss der Anpressdruck erhöht werden, um den Übergang der Ultraschallwellen vom Sender auf den Probekörper und Empfänger fehlerfrei sicherzustellen. Ein zu geringer Anpressdruck verfälscht die Messung der Resonanzfrequenz.

Aus Figur 4 kann weiterhin die Schlussfolgerung gezogen werden, dass ab einer Anpresskraft von etwa 14 N die Verstärkung konstant bleibt und dadurch der Fehler der Resonanzfrequenzmessung minimiert wird. Die Streuung reduziert sich ab der Anpresskraft von 14 N auf 0,01 %.

Wie bereits ausgeführt, werden die Formkörper einem Trocknungsverfahren unterworfen, um die Gewichtskonstanz nach der Auslagerung der Prüfkörper sicherzustellen. Anhand der ermittelten Zeiten zum Erreichen der Gewichtskonstanz wurde der Einfluss unterschiedlicher verbleibender Oberflächenfeuchten auf die Bestimmung Resonanzfrequenz und dem daraus berechneten Elastizitätsmodul ermittelt. In der nachstehenden Tabelle 2 sind die Ergebnisse der Untersuchungen aufgelistet. Nach dem Abtropfen des anhaftenden Oberflächenwassers für eine Dauer von 45 Sekunden unter einer Neigung und Drehung von 45° verblieb eine Oberflächenfeuchte von durchschnittlich 0,13 %. Bei gleicher Neigung und Drehung unter Einwirkung eines Druckluftstrahles für eine Dauer von 10 Sekunden verblieb 0,05 % anhaftendes Wassers.

EP 1 211 511 B1

Tabelle 2:

| Vergleichende Resonanzfrequenzmessungen an oberflächentrockenen und oberflächenfeuchten Prismen nach Abtropf- bzw. Abblasverfahren | | | | | | | | | |
|---|---|---|---|---|---|---|---|---|---|
| Nr. | trockenes Prisma | | | Abgetropftes Prisma | | | Abgeblasenes Prisma | | |
| | Masse | $n_D$ | $E_D$ | Masse | $n_D$ | $E_D$ | Masse | $n_D$ | $E_D$ |
| 1 | 602,6 | 14602 | 51394 | 603,4 | 14570 | 51237 | 602,9 | 14597 | 51385 |
| 2 | 602,6 | 14617 | 51500 | 603,4 | 14578 | 51293 | 602,9 | 14607 | 51455 |
| 3 | 602,6 | 14601 | 51387 | 603,4 | 14588 | 51364 | 603 | 14612 | 51499 |
| 4 | 602,6 | 14615 | 51486 | 603,3 | 14573 | 51250 | 602,9 | 14598 | 51392 |
| 5 | 602,6 | 14610 | 51950 | 603,3 | 14592 | 51383 | 602,8 | 14612 | 51482 |
| MW | 602,6 | 14609 | 51543,4 | 603,36 | 14580 | 51305 | 602,9 | 14605,2 | 51442,6 |
| s | 0,0 | 7,3 | 233,1 | 0,1 | 9,5 | 65,9 | 0,1 | 7,3 | 51,9 |
| s [%] | 0,00 | 0,05 | 0,45 | 0,01 | 0,07 | 0,13 | 0,01 | 0,05 | 0,10 |

Es kann die Schlussfolgerung gezogen werden, dass mit zunehmender Oberflächenfeuchte die Resonanzfrequenz und der E-Modul sinkt. Unter Berücksichtigung der ermittelten Geräteschwankungen können zwischen Abblasen der Prismen mittels Druckluft und den vollständig abgetrockneten Prismen nur Unterschiede festgestellt werden, die innerhalb der Schwankungsbreite des Geräte liegen.

Neben den Standardprüfterminen im Alter von 1, 2, 7 und 28 Tagen ist auch die Festigkeitsentwicklung zu frühen Zeitpunkten von Interesse. Hierzu werden Normmörtelprüfkörper hergestellt und zu verschiedenen Zeitpunkten entformt. Der Zeitpunkt zum Entformen der Probekörper wird zu Beginn mit etwa der doppelten Zeitspanne des Erstarrungsendes angesetzt. Nach der Messung der Grundresonanzen wurde das Verhältnis dieser zueinander berechnet. In Figur 6 sind beispielhaft die Messungen an CEM I Zementen verschiedener Werke in Abhängigkeit des Probenalters dargestellt. Die Werte in Bereich 0 - 10 Stunden liegen teilweise sogar oberhalb von 0,60. Ein möglicher Grund hierfür ist die sich noch stark ändernde Zementmatrix.

Der markierte Richtwert von 0,58 wird bei der Messung der Grundresonanzen als korrektes Verhältnis zwischen der Dehnungs- und Torsionsresonanzfrequenz betrachtet. Aufgrund der durchgeführten Messungen ergibt sich für das Verhältnis der Grundresonanzen zueinander eine Schwankungsbreite von ± 0,005 um den Richtwert von 0,58. Das Mindestprobenalter wird durch das Erreichen dieses Zielkorridors charakterisiert. Erst wenn das Probenalter in etwa dem dreifachen der Zeit des Erstarrungsendes erreicht, nähert sich das zur Kontrolle der Messungen dienende Verhältnis dem Richtwert von 0,58 ± 0,005. Nach der Messung an Zementen verschiedener Festigkeitsklassen der Arten CEM I, CEM II und CEM III kann das Mindestalter zur korrekten Bestimmung der Druckfestigkeiten mittels Ultraschallresonanzfrequenzverfahren etwa mit dem 3,5 -fachen der Zeit des Erstarrungsendes angegeben werden.

Die mit Hilfe der erstellten Formeln berechneten Druckfestigkeiten weichen zu diesen sehr frühen Terminen zwischen 5 und 18 % von der zerstörend ermittelten Druckfestigkeit ab. Die maximale Abweichung mit 18 % wurden bei einem CEM III B 32,5 NW HS ermittelt. Die berechneten Druckfestigkeiten lagen 0,5 N/mm$^2$ über den gemessenen Druckfestigkeiten von 2,7 N/mm$^2$. Die Höhe der Abweichungen verringert sich mit zunehmendem Probenalter und steigender Druckfestigkeit. Im Bereich der 1-Tages-Druckfestigkeiten reduziert sich diese Abweichung auf Werte kleiner 5 % je nach Zementsorte.

Mit Hilfe der Resonanzfrequenzmethode wurden eine Reihe von verschiedenen Zementen aus verschiedenen Produktionsstätten hinsichtlich der Korrelation der Druckfestigkeit und des dynamischen Elastizitätsmoduls untersucht.

Die Prüfung wurde nach der DIN EN 196-1 durchgeführt. Die Prüfzeitpunkte sind mit 1, 2, 7 und 28 Tagen festgelegt worden. Die nachstehende Tabelle 3 zeigt die Anzahl der untersuchten Zemente getrennt nach Arten.

Tabelle 3:

| Untersuchte Einzelproben der I, II und III getrennt nach Zementarten | | | |
|---|---|---|---|
| Werk | CEM I | CEM II | CEM III |
| I | 2 | 1 | 2 |
| II | 1 | 2 | -- |
| III | 4 | 1 | 2 |

Zur Überprüfung des Verfahrens hinsichtlich der Eignung bei auftretenden Produktionsschwankungen wurden die Messungen auf einen Zeitraum von 2 bzw. 4 Wochen ausgedehnt. Die Untersuchungen wurden in den Werken VI und IV über einen Zeitraum von vier und im Werk V für die Dauer von 2 Wochen durchgeführt. Die Prüftermine richteten sich hierbei nach dem Prüfplan der jeweiligen Werke. Die nachstehende Tabelle 4 zeigt die Anzahl der untersuchten Zemente nach Art und Werk getrennt.

Tabelle 4:

| Zemente der untersuchten Werke IV, V und VI getrennt nach Zementarten | | | |
|---|---|---|---|
| Werk | CEM I | CEM II | CEM III |
| IV | 5 | 3 | 2 |
| V | 3 | 1 | -- |
| VI | 7 | 3 | 1 |

Neben der Bestimmung von Masse und Grundresonanzen wurden auch die Kenngrößen der RRSB-Verteilung als auch die unterschiedlichen Klinkerphasengehalte protokolliert. Bei den Kompositzementen wurden Art und Gehalt der Zumahlstoffe festgehalten. Alle Kenngrößen sollen durch weitergehende Analyse zur Verfeinerung der Fittfunktion dienen.

Aufgrund der geringen Schwankungen bei den Prüfkörperabmessungen wurde auf eine genaue Bestimmung der Prüfkörperabmessungen verzichtet. Für die Berechnung der Prüfrohdichte wurden diese mit 16x4x4 cm festgesetzt.

Im Nachfolgenden wird der Gang der Auswertung bis hin zur Auswertung der Ergebnisse beschrieben, beginnend mit der Vorgehensweise zum Auffinden der Grundresonanzen.

Je nach zu bestimmender Resonanzfrequenz wird der Probekörper entsprechend eingespannt. Hierbei ist auf einen ausreichend hohen Anpressdruck zu achten. Die Frequenz wird solange verändert bis sich die Lissajous-Figuren im Oszilloskopen drehen. Die Figur ist im Bereich der Resonanzfrequenz nur noch als Strich zu sehen. Die Anzeige der Verstärkung des Messsignals zeigt im Bereich der Resonanzfrequenz nun ein Maximum. Dieses ist mit Hilfe der Verstärkung von Aufnehmer und Geber von mindestens 60 % einzustellen, um Messfehler zu vermeiden. Das Gerät zeigt die ermittelte Resonanzfrequenz in der Digitalanzeige des Geräts. Anhand der ermittelten Dehnungsresonanz lässt sich über das Verhältnis von 1:0,58 die Torsionsresonanzfrequenz vorausberechnen.

Nach der Bestimmung der Prüfkörpermasse und der Resonanzfrequenzen erfolgt die Berechnung der Prüfrohdichte. Anhand der Prüfkörpermaße, der Prüfrohdichte und der Resonanzfrequenzen werden auf der Basis der nachstehenden Beziehungen das Dehnungs- und Schubmodul berechnet.

Berechnung des dynamischen Elastizitätsmoduls $E_D$:

$$E_D = 4\ L^2\ n_D^2\ \rho$$

| $E_D$ | dynamischer E-Modul | [N/mm$^2$] |
|---|---|---|
| L | Länge | [m] |
| $n_D$ | Dehnungsresonanzfrequenz | [kHz] |
| $\rho$ | Rohdichte | [kg/m$^3$] |

Berechnung des Torsions- oder Schubmoduls $G_T$:

$$G_T = 4\ k\ L^2\ n_T^2\ \rho$$

| $G_T$ | Schubmodul | [N/mm$^2$] |
|---|---|---|
| k | Faktor k = 1,183 für Balken mit quadratischem Querschnitt | |
| L | Länge | [m] |
| $n_T$ | Torsionsresonanzfrequenz | [kHz] |
| $\rho$ | Rohdichte | [kg/m$^3$] |

Der weitere Gang der Auswertung zur Berechnung der Druckfestigkeit wird nachfolgend beschrieben. Mit Hilfe der berechneten Druckfestigkeit kann dann die Biegezugfestigkeit errechnet werden.

Die Auswertung der berechneten E-Moduli und der zerstörend gemessenen Biegezug- und Druckfestigkeiten wurde mit Hilfe einer Elektronischen Datenverarbeitung vorgenommen. Die gewählten Programme sollen die Möglichkeit bieten, Korrelationen anhand gewählter Funktionen anzufitten. Die Optimierung der Variablen erfolgt über den chi$^2$-Test und das Bestimmtheitsmaß R$^2$. In weiteren Schritten wurde versucht, aufgrund der zusätzlich dokumentierten Daten der Klinkerchemie und Kornverteilung die formelmäßige Abhängigkeit weiter zu verfeinern.

Nach Analyse verschiedener Fittfunktionen über das Bestimmtheitsmaß und den chi$^2$-Test wurden alle untersuchten Zemente abweichend zur im Stand der Technik aufgestellten potentiellen Abhängigkeit mit Hilfe nachstehender Funktion angefittet:

$$y = a\, e^{b\,x}$$

Die hierdurch entstehende Formel ist nur für den jeweils zugrundeliegenden Zement und für den Prüfbereich zwischen 1 und 28 Tagen gültig. Im Bereich der Frühfestigkeiten und Festigkeiten über die 28 Tage hinaus kann die gleiche Formel verwendet werden, dabei muss je nach Zement jedoch mit teilweise größeren Abweichungen zu den zerstörend bestimmten Druckfestigkeiten gerechnet werden. Anhand des nachstehenden Diagramms soll verdeutlicht werden, dass diese Korrelationsuntersuchung zum derzeitigen Zeitpunkt für jeden Zement einzeln durchgeführt werden muss. Das Diagramm zeigt Zemente der Sorte CEM I 32,5 R verschiedener Werke.

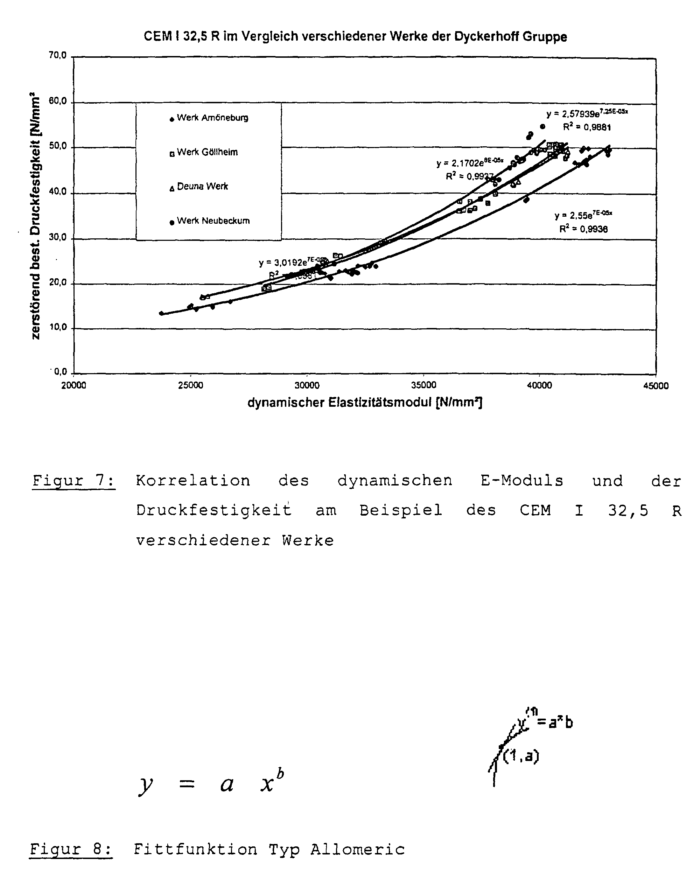

Anhand von Figur 7 wird deutlich, dass, obwohl die Zemente der Werke I, IV und V bis zu einem dyn. E-Modul von 30.000 N/mm$^2$ eng zusammenliegen, sich diese im weiteren Erhärtungsverlauf und der damit verbundenen E-Modulbestimmung unterscheiden.

Anhand der Korrelationsuntersuchungen konnte ein Katalog an Variablen zusammengestellt werden, mit dessen Hilfe die Druckfestigkeit aus den Werten des dyn. E-Moduls für jeden einzelnen Zement berechnet werden kann.

Die Biegezugfestigkeit kann anhand der Druckfestigkeit über eine Potenzfunktion berechnet werden. In Figur 8 wird die Funktion und deren graphisches Abbild dargestellt.

Die Werte für die Variable a müssen immer größer als 0 sein und die Werte für die Variable b liegen im Bereich zwischen 0,5 und 0,7. In einigen wenigen Ausnahmefällen konnten auch Werte außerhalb dieses Bereiches ermittelt werden. Anhand der formelmäßigen Abhängigkeit und den ermittelten Variablen können die Biegezugfestigkeiten aus der Druckfestigkeit der einzelnen Prüfkörper berechnet werden.

Zur Überprüfung der Übereinstimmung der zerstörungsfrei und der zerstörend ermittelten Druckfestigkeiten wurden die Mittelwerte und Standardabweichungen der geprüften Prismensätze berechnet. An dieser Stelle soll beispielhaft für das Werk IV die Korrelation der zerstörungsfrei gegenüber den zerstörend bestimmten Druck- und Biegezugfestigkeiten aufgezeigt werden.

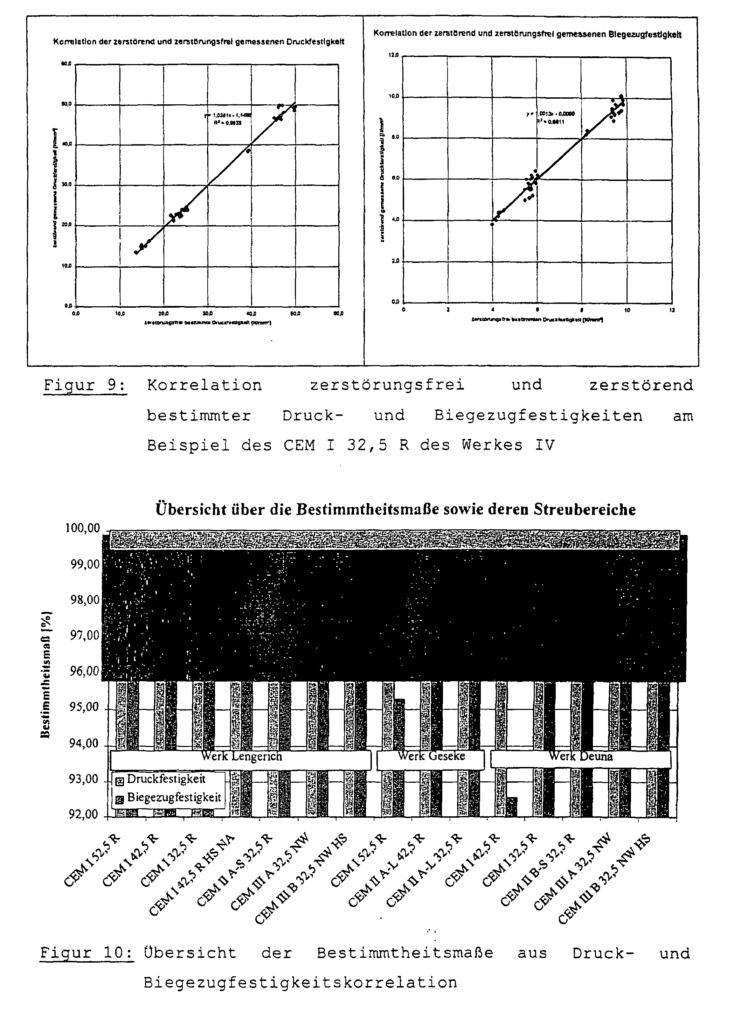

Alle untersuchten Zemente wurden in gleicher Art und Weise ausgewertet (siehe Figur 9).

Die Beurteilung der Korrelationsuntersuchungen charakterisiert die Güte des Verfahrens zur Ermittelung der Biegezug- und Druckfestigkeiten. Darüber hinaus kann eine Aussage getroffen werden, inwiefern die über einen längeren Zeitraum auftretenden Produktionsschwankungen einen Einfluss auf die Güte des Verfahrens nehmen.

Nach der Auswertung aller Einzelproben und der Gegenüberstellung der zerstörungsfrei und zerstörend ermittelten Festigkeiten sind die Bestimmtheitsmaße in Figur 10 zusammengestellt worden. Die linken vertikalen Balken geben das Bestimmtheitsmaß des jeweiligen Zementes für die Übereinstimmung der Druckfestigkeiten an und die rechten vertikalen Balken stehen für die Übereinstimmung der Biegezugfestigkeit. Die transparent angedeuteten Rechtecke charakterisieren die zugehörigen Streubereiche.

Auf der Basis von Figur 10 lässt sich eine sehr gute Übereinstimmung zwischen der Druckfestigkeitsprüfung nach DIN EN 196-1 und der zerstörungsfrei ermittelten Druckfestigkeit mittels der Ultraschallresonanzfrequenzmethode feststellen. Das Bestimmtheitsmaß R$^2$ für die Korrelation der Druckfestigkeiten liegt im Mittel mit einer Streuung bei 99,73 $\pm$ 0,26.

Nach der Auswertung aller Einzelproben ist anhand der erfindungsgemäß gewählten Exponentialfunktion für alle Zemente der verschiedenen Werke eine sehr gute Korrelation zwischen der aus dem dynamischen Elastizitätsmodul berechneten und der zerstörend ermittelten Druckfestigkeit nachgewiesen worden. Die Abweichung zwischen der berechneten und der zerstörend bestimmten Druckfestigkeit liegt zu den jeweiligen Prüfterminen durchschnittlich bei einer Abweichung von weniger als 3 %. Die Schwankungen zwischen zwei Prismenhälften bezogen auf den Mittelwert zweier Prismenhälften beträgt durchschnittlich 2 %. Die maximale Schwankung zwischen zwei Hälften bezogen auf den Mittelwert konnte mit 8,7 % festgestellt werden. Im Bereich der Standardabweichung des Primensatzes liefern beide Verfahren vergleichbare Ergebnisse. Aufgrund der mit der Erfindung erzielten sehr guten Korrelation und der vergleichbaren Streuungen wird die Schlussfolgerung gezogen, dass die erfindungsgemäße Bestimmung der Druckfestigkeit mittels des erfindungsgemäßen Verfahrens als äquivalentes Verfahren zur Druckfestigkeitsprüfung nach DIN EN 196 Teil 1 gesehen werden kann. Demgegenüber weist das erfindungsgemäße Verfahren jedoch erhebliche Vorteile auf.

Die Korrelation der Biegezugfestigkeiten liegt mit Werten für das $R^2$ von 97,85 ± 2,01 etwas schlechter. Die Standardabweichungen der Biegezugfestigkeitsprüfung liegen deutlich über denen der Biegezugfestigkeitsberechnung. Das geringe Bestimmtheitsmaß wird jedoch im wesentlichen durch die hohen Streuungen der zerstörenden Biegezugprüfung hervorgerufen. Die Streuung nimmt mit steigenden Festigkeiten zu. Trotz der guten Übereinstimmung der Korrelation mit 97,85 wichen Einzelwerte aufgrund der genannten Streuung der Biegezugprüfung 15 % voneinander ab. Die Varianz oder bezogene Streuung liegt mit durchschnittlich 8 % deutlich über der Varianz der berechneten Biegezugfestigkeiten, die bei etwa 1-2 % liegt. Hinsichtlich der Streuung der Biegezugfestigkeiten lassen sich mit der Berechnung der Biegezugfestigkeiten aus den Druckfestigkeiten bessere Ergebnisse erzielen als mit der herkömmlichen Biegezugprüfung.

Dass die zerstörungsfreie Bestimmung der Druckfestigkeit mittels Ultraschall gute Ergebnisse liefert wurde bereits durch Catharin in den Jahren 1961 und 1963 gezeigt. Damals wurde versucht, Formeln aufzustellen, mit deren Hilfe die Berechnung aufgrund der bekannten Zementchemie und -feinheit vorgenommen werden kann. Es konnte im Rahmen der Erfindung herausgefunden werden, dass sowohl Zementchemie als auch die Feinheit des Zementes einen Einfluss auf den Verlauf der Funktion haben. Im Gegensatz zu Catharin werden erfindungsgemäß die Kenndaten der RRSB-Verteilung und nicht die spezifische Oberfläche nach Blaine verwendet. Bei Kenntnis, der erwähnten Kenndaten sollen die Variablen der Exponentialfunktion vorausberechnet werden. Ausgangspunkt der Untersuchungen sind die vier Hauptklinkerphasen Tricalciumsilikat, Dicalciumsilikat, Tricalciumaluminat und Tetracalciumaluminatferrit sowie die Kenndaten der RRSB-Kornverteilung aus der Lasergranulometrie der Lageparameter und das Steigungsmaß.

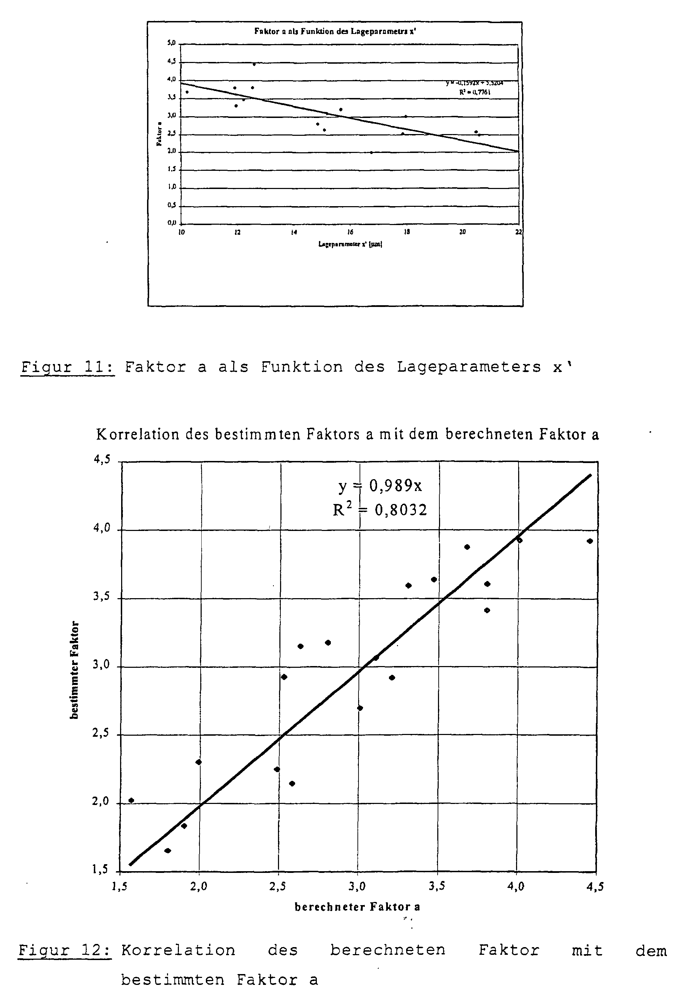

Anhand der durchgeführten Korrelationsuntersuchungen entstand der Eindruck, dass der Faktor im wesentlichen durch den Lagerparameter x' beeinflusst wird. Die Korrelation des Lageparameters mit dem bestimmten Faktor a soll anhand von Figur 11 aufgezeigt werden.

Für die weitere Optimierung der Berechnung des Faktors a wurden die Daten des Lageparameters, Tricalciumsilikates und des Tricalciumaluminates verwendet. Da diese genannten Faktoren gerade zu frühen Zeitpunkten die Erhärtung beeinflussen konnte die Korrelation des berechneten Faktors und des bestimmten Faktors weiter gesteigert werden. Nachfolgend wird die ermittelte Formel zur Berechnung des Faktors a und in Figur 12 das Korrelationsdiagramm der Übereinstimmung zwischen der Berechnung und der Bestimmung des Faktors a gezeigt.

Formel zur Berechnung des Faktors a:

$$a = 1,56607 - 0,158809 * x' + 0,766436 * n$$

$$+ 0,0352516 * C_3S + 0,110678 * C_3A$$

Über die Berücksichtigung der Klinkerchemie ($C_3S$ und $C_3A$) konnte das Bestimmtheitsmaß auf 0,8032 gesteigert werden. Aufgrund der zu geringen Übereinstimmung sollte die hier erstellte Formeln nur zur Startpunktsabschätzung der Fittfunktion für den Faktor a verwendet werden.

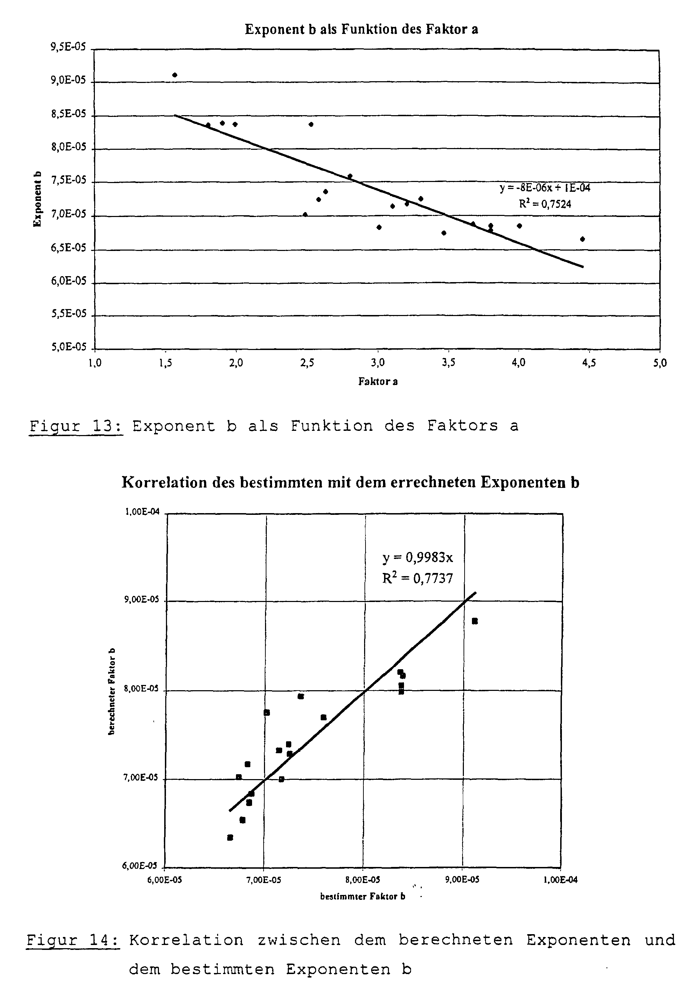

Der Exponent b beschreibt im wesentlich die Steigung der Exponentialfunktion. Der Faktor a charakterisiert bei einem Exponenten vom Wert 0 den Startpunkt der Funktion, somit hat der Faktor a einen wesentlichen Einfluss auf die Berechnung des Wertes b, was auch mit Hilfe von Figur 13 verdeutlicht werden kann.

Die Einflussfaktoren, die maßgeblich auch die Erhärtung beeinflussen müssen zwangsläufig auch die Berechnung des Exponenten b beeinflussen. Zu diesen Größen zählen die Klinkerphasen Tricalciumsilikat, Dicalciumsilikat und Tricalciumaluminat sowie die Kenngrößen der RRSB-Verteilung der Lageparameter und das Steigungsmaß. Durch Analyse der genannten Daten kann eine Formel zur Berechnung des Exponenten sowie ein Korrelationsdiagramm (siehe Figur 14) des berechneten gegenüber dem bestimmten Exponenten b ermittelt werden.

Formeln zur Berechnung des Exponenten b:

$$b = 1,31478 \; 10^{-4} - 1,10735 * 10^{-5} * a$$

$$- 6,2349 * 10^{-7} * x' - 2,15466 * 10^{-7} * C_3S$$

$$- 5,66977 * 10^{-7} * C_2S + 9,60419 * 10^{-7} * C_3A$$

Durch die Berücksichtigung der Klinkerchemie und der Kenndaten der RRSB-Kornverteilung konnten die Korrelation ausgedrückt durch das Bestimmtheitsmaß von 0,6714 auf 0,7737 gesteigert werden. Auch hier gilt die Aussage, dass die Formel aufgrund der geringen Übereinstimmung nur zur Festlegung des Startpunktes der Fittfunktion herangezogen werden sollte.

Der hier aufgezeigte Weg ist eine Möglichkeit sein den Faktor und den Exponenten an die auftretenden Produktionsschwankungen anzupassen. Über diese Form der Faktorenkorrektur werden weitere Korrelationsuntersuchungen reduziert bzw. die Zeiträume zur Überprüfung der vorgegebenen Berechnungsgrundlagen verlängert.

Nach der Angabe geeigneter Probenkennzeichnungssysteme, der Charakterisierung verschiedener Einflussgrößen auf die Messung der Grundresonanzen sowie der Darstellung der Korrelation zur zerstörungsfreien Ermittlung der Druck- und Biegezugfestigkeiten wird nachfolgend zusammengefasst der Aufbau der Prüfstandsapparatur wiedergegeben. Ferner werden die Maßnahmen zur Umsetzung dieser Konzeption aufgegriffen. Die Maßnahmen beinhalten die Lösungen zu der Problemstellung des Transportes und auch weitere Maßnahmen zur umfangreicheren Nutzung und Anwendung des Prüfstandes für andere mineralische Bindemittelsysteme.

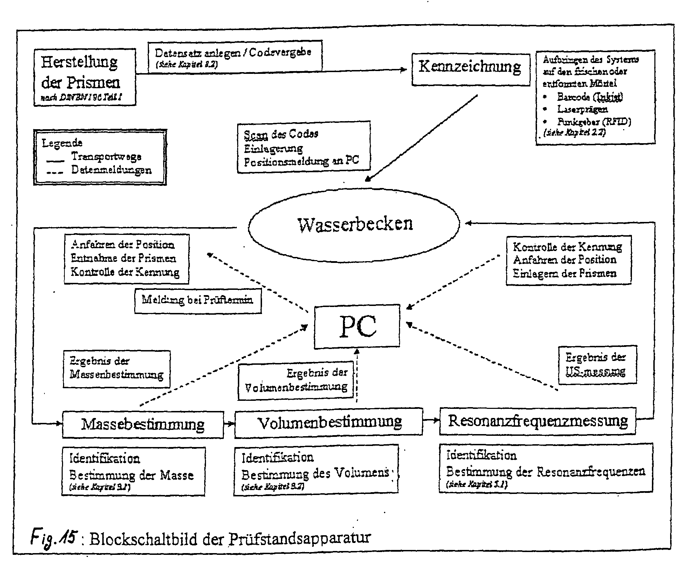

Zur Veranschaulichung der erfindungsgemäßen Prüfstandsvorrichtung soll das Blockschaltbild gem. Fig. 15 dienen.

Das Blockschaltbild zeigt den Weg der Prüfkörper ab dem Zeitpunkt der Herstellung. Nach der Kennzeichnung mittels des RFID-Systems erfolgt die Einlagerung der Prüfkörper. Mit der Einlagerung wird auch die Lagerposition der Prismen den Datensatz zugeordnet. Zum jeweils vorgegebenen Prüftermin wird der dargestellte Prüfzyklus bestehend aus Auslagerung, Bestimmung von Masse, Volumen und Grundresonanzen und erneuter Einlagerung abgearbeitet.

Weitere Punkte die zur Umsetzung des Prüfstandes nutzvoll erscheinen, sind nachfolgend festgehalten.

Bei der Herstellung der Prüfkörper ist darauf zu achten, dass Formen mit glatten Kopfflächen und nicht die mit Einkerbungen für die Zapfen der Schwindmessung verwendet werden. Glatte Prüfflächen erleichtern das Ankoppeln der Prüfköpfe. Nach dem Entformen der Prüfkörper sind diese mit den codierten Datenträgern zu bekleben. Die Datenträger sollten mittig auf die Prismenoberfläche geklebt werden, da sich in der Mitte des Prüfkörpers auch der Schwingungsknoten ausbildet. Im Anschluss an die Kennzeichnung erfolgt das Einlesen der Codierung und das Anlegen des Datensatzes für die Protokollierung aller Ergebnisse in Abhängigkeit des Probenalters. Die gekennzeichneten Prüfkörper werden auf die bereits erwähnten erfindungsgemäßen Kunststoffprofile gelegt (Figur 15, Bezugszeichen 1) und dem Robotersystem zur Einlagerung bereitgestellt (Figur 15, Bezugszeichen 2).

Das Robotersystem dient im wesentlichen zur Ein- und Auslagerung der Prüfkörper. Hierbei kann das System selbständig aufgrund der programmierten Taktzeiten entscheiden, ob genügend Zeit zur Einlagerung besteht oder ob Prüfkörper zum nächsten Prüftermin ausgelagert werden sollen. Für den Fall der Einlagerung identifiziert der Roboter mit Hilfe einer am Greifarm montierten Antenne die am Prüfkörper angebrachten Datenträger. Über eine Lagerplatzverwaltung erkennt das System freie Lagerplätze und kann die Einlagerung vornehmen.

Bei der Einlagerung sind nachstehende Punkte mit Hilfe der Software zur Lagerplatzverwaltung zu realisieren:

- Ein Lagerort beinhaltet drei Lagerplätze für drei Prüfkörper

- Lagerplätze müssen für die Zeit des gewählten Prüfzeitraumes gesperrt bleiben

- Bei Lagerung in großen Wasserbecken ist auf die Trennung nach Zementarten zu achten

- Nach Ablauf des gewählten Zeitraumes sind die Prüfkörper nicht wieder einzulagern

- Nach Ablauf des gewählten Zeitraumes sind die Lagerplätze automatisch freizugeben

Für die Lagerung der Prüfkörper lassen sich zwei Varianten verwenden. Bei den zumeist bestehenden Wasserbecken ist die Linearachse an die Länge der Wasserbecken anzupassen. Bei neu konzipierten Prüfständen sollte zur besseren Raumausnutzung ein klimatisierter Klimaschrank im Paternosterverfahren eingesetzt werden. Hierbei können kleine Wasserbecken für die Lagerung eines einzelnen Satzes eingesetzt werden. Bei der letztgenannten Variante entfällt die Kontrolle der getrennten Lagerung nach Zementsorten, da jeweils nur ein Satz in die kleinen Becken eingelagert werden kann.

Die Koordinaten der Lagerplätze sind in dem zugehörigen Datensatz abzuspeichern. Diese Koordinaten dienen bei Erreichen des Prüftermins zum Anfahren der Prüfkörperposition durch den Roboter (Figur 15 Nr. 2). Über die am Roboterarm montierte Antenne erfolgt die Verifizierung zwischen zu prüfendem Prisma und der angefahrenen Position.

Die Software muss in der Lage sein, die Prüfzeiten in Abhängigkeit des Prüfalters gemäß DIN EN 196 Teil 1 einzuhalten. Hierbei ist die Zeit für Auslagerung, Transport und Messung der Prüfgrößen zu beachten. Die nachstehende Tabelle 5 listet die zulässigen Abweichungen der Prüfzeit in Abhängigkeit des Probenalters auf.

Tabelle 5:

| zulässige Abweichungen der Prüfzeit je nach Prüfalter | |
|---|---|
| Prüfalter [d] | Zulässige Abweichung |
| 1 | +/- 15 min |
| 2 | +/- 30 min |
| 3 | +/- 45 min |
| 7 | +/- 2 h |
| >28 | +/- 8 h |

Die Prüfkörper dürfen 20 Minuten vor Prüfbeginn aus dem Wasserbecken entnommen werden. Nach dem Herausheben aus dem Lagerungsbecken erfolgt das Abtropfen bzw. Abblasen bis zur Gewichtskonstanz auf Basis der erstellten Kurven. Durch erfindungsgemäß zusätzlich am Roboterarm montierte Druckluftdüsen werden die Prismen vorzugsweise unter einer Neigung von 45° sowie einer Drehung um 45° um die Längsachse für 10 Sekunden mit einem Druckluftstrahl von 2 bar abgeblasen.

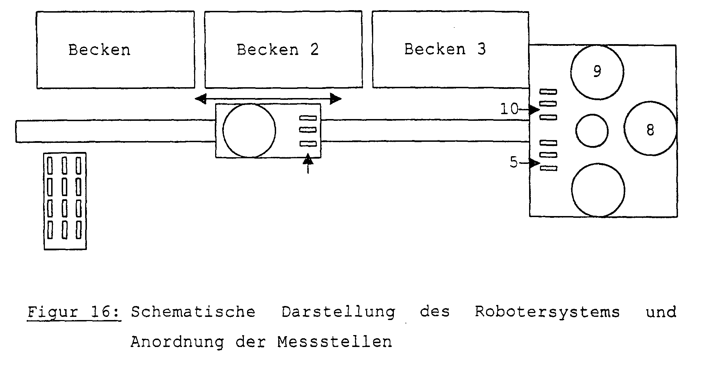

Nach dem Auslagern und Abblasen werden die Prismen auf Zwischenlagerplatz 4 am verfahrbaren Robotersystem 2 zwischengelagert (Fig.16). Der Zwischenlagerplatz 4 besteht aus drei Plätzen für drei Prüfkörper, so dass immer der komplette Satz zum Prüftermin ausgelagert werden kann. Am Ende der Linearachse 3 befinden sich die kreisförmig angeordneten drei Messplätze 7, 8, 9. Vor den drei Messstellen befinden sich zwei Kunststoffprofile mit je drei Lagerplätzen 5, 10. Auf das erste Kunststoffprofil 5 erfolgt die Anlieferung der zu prüfenden Prismen, auf dem zweiten Kunststoffprofil 10 lagern die bereits geprüften Prismen, bereit zur erneuten Einlagerung bis zum nächsten Prüftermin. Die drei Messstellen dienen zur Bestimmung der Masse 7, der Prüfkörperdimensionen 8 und zur Messung der Grundresonanzen 9. Die Ergebnisse der Messungen sind in Abhängigkeit vom Probenalter zu archivieren. Die Berechnung der Druck- und Biegezugfestigkeit erfolgt aufgrund der in einer Datenbank hinterlegten zementspezifischen Formeln. Ein zweiter kleinerer Roboter 6 entnimmt die Prüfkörper vom ersten Profil 5 und führt diese den drei Messplätzen zu und legt die Prismen nach den Messungen auf das zweite Profil 10.

Die Bestimmung der Masse bereitet keinerlei Schwierigkeiten und hat die kürzesten Taktzeiten. Die Ermittlung der Abmessungen kann bei Einsatz im Routinebetrieb mit induktiven Wegaufnehmer mit einer Genauigkeit von ± 0,1 mm erfolgen. Für anspruchsvollere Untersuchungen der Volumendehnung sind Wegaufnehmer mit einer Genauigkeit von bis zu ± 0,001 mm oder ein Lasersystem auf Grundlage der Lasertriangulation mit einer Genauigkeit von ± 0,01 mm zu verwenden. Mit steigender Genauigkeit und Auflösung der Vermessung erhöht sich die Taktzeit der Bestimmung. Des weiteren erhöht sich auch der Preis des Systems.

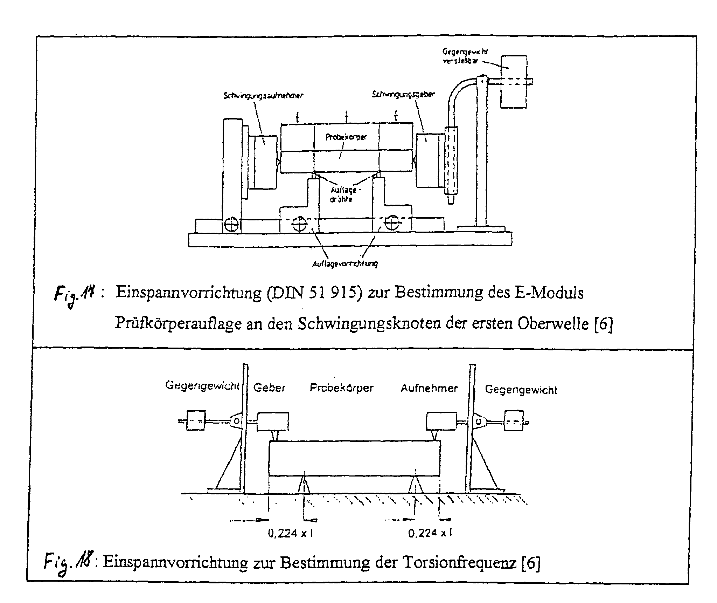

Bei der Bestimmung der Grundresonanzen bietet sich die Verwendung von zwei EG-Metern mit entsprechenden Prüfköpfen und Stativen an. Der Vorteil von zwei Geräten ist die Einsparung einer aufwendigen Einrichtung, die in der Lage ist, beide Varianten der Ankopplung zur Messung der Dehnungs- und Torsionsresonanzfrequenz zu realisieren. Weiterhin kann auf diese Art und Weise die Taktzeit an dieser Messstelle verkürzt werden. Die Figuren 17 und 18 zeigen mögliche Einspannvorrichtungen zur Messung der jeweiligen Resonanzfrequenz.

Die dargestellten Einspannvorrichtungen sind mit Positionierungshilfen auszustatten, die dafür sorgen, dass die Prismen immer exakt gemäß den Abmessungen in Bild 8.4 und 8.5 eingespannt sind. Diese Justierung sorgt dafür, dass die Schwingungsknoten der Ultraschallwellen mit den Auflagerpunkten der Prüfkörper übereinstimmen. Zum Schutz der Mitarbeiter vor den durch die Resonanzmessungen erzeugten hörbaren Schallwellen ist diese Prüfstation schalldicht einzukapseln.

Zur Kontrolle der Resonanzfrequenzmessung dient das Verhältnis der Dehnungs- und Torsionsfrequenz von 1:0,58. Die grobe Einstellung der Resonanzfrequenz in Abhängigkeit des Prüfalters kann mit Hilfe der erfindungsgemäß gewonnenen Daten erfolgen. Die Feinjustierung zur Ermittlung der Resonanzfrequenz muss mit Hilfe der Verstärkung automatisiert werden. Im Bereich der Resonanzfrequenz erreicht die Verstärkung ein Maximum. Zur korrekten Bestimmung der Grundresonanzen ist über eine Kontrolleinheit darauf zu achten, dass die Verstärkung bei Erreichen der Resonanzfrequenz mindestens 60 % überschreitet. Aufgrund unterschiedlicher Dämpfung je nach Zement und Alter kann die Verstärkung trotz ausreichenden Anpressdruckes der Prüfköpfe zu gering sein, so dass die Messung fehlerhaft abläuft. Um solche Fehler auszuschließen, sollte die Verstärkung vom Geber auf Schalterstellung 3 gestellt werden und die Stellung vom Aufnehmer erhöht werden bis die Verstärkung mindestens einen Wert von 60 % im Bereich der Resonanzfrequenz erreicht. Nach Beendigung des Prüfzyklus erfolgt die Wiedereinlagerung der Prüfkörper bis zum nächsten Prüftermin. Nach Beendigung des letzten Prüfzyklus zum Ende des festgelegten Prüfzeitraumes werden die Prüfkörper zurück an die Position 1 gefahren. Zum Abschluss kann zur Überprüfung der errechneten Festigkeiten die Festigkeit nach DIN EN 196-1 oder die Entsorgung der Prüfkörper erfolgen.

Es sollen nachfolgend beispielhaft die wichtigsten Inhalte eines Datensatz erfasst und aufgelistet werden. Nach dem Anlegen eines Datensatzes werden die Datensatzfelder während des Prüfzeitraumes mit den Messwerten gefüllt werden. Zu jedem Prüftermin erfolgt eine Messwertkontrolle. Die ermittelten Prüfgrößen Masse, Volumen und Grundresonanzen bzw. Verhältnis der Grundresonanzen sollen mittels einer Onlinekontrolle anhand von vorgegebenen Grenzwerten überprüft werden. Bei einer Überschreitung der eingestellten Streubereiche ist eine Warnmeldung auszusenden. Die nachstehende Tabelle 6 listet unverzichtbare Datenbankinformationen und deren Funktion auf.

Tabelle 6:

| Datensatzfelder und anwählbare Funktionen zum Anlegen eines Datensatzes | | |
|---|---|---|
| Information | Funktion | Bemerkung |
| Labornummer | Eingabe durch den Laboranten | Möglichkeit zur Verknüpfung mit anderen Datenbanken |
| Code-Kennung | Im Datenträger fest eingebrannt Aufnahme in den Datensatz über die Antenne | Identifikation der Prüfkörper an den jeweiligen Messplätzen |
| Herstellungsdatum Herstellungszeit | Eingabe durch den Laboranten | Berechnung der gewählten Prüftermine |
| Zementsorte Hersteller Werk | Anwählbar durch vorgegebene Produktpalette | Angaben dienen zur Auswahl hinterlegter Richtgrößen und Variablen zur Berechnung der Festigkeiten |
| Prüftermine | Mehrere Auswahlmöglichkeiten 1, 2, 7, 28, 56 Tage sowie frei wählbare Zusatztermine | Berechnung der Prüftermine aufgrund von Herstelldatum und -zeit Abschätzung der Grundresonanzen in Abhängigkeit der Zeit |
| Lagerplatzkoordinaten | Rückmeldung der Koordinaten nach Einlagerung | Anfahrpositionen des Roboters zur Ein- und Auslagerung der Prismen |

Über den gesamten Prüfzeitraum sind die ermittelten Daten zu erfassen und abzuspeichern. Dafür sind in entsprechende Tabellen je Prüftermin folgende Felder vorzusehen:

- Masse der Prüfkörper m

- Abmessungen der Prüfkörper l x b x h

- Berechnete Prüfrohdichte der Prüfkörper $\rho$

- Dehnungsresonanzfrequenz $n_D$

- Torsionsresonanzfrequenz $n_T$

- Berechnetes dynamisches Elastizitätsmodul $E_D$

- Berechnetes dynamisches Schubmodul $G_T$

- Berechnete Poission'sche Konstante $\mu$

- Berechnetes Verhältnis der Grundfrequenzen $n_D : n_T$

- Berechnete Druck- und Biegezugfestigkeit je Prüfkörper

- Mittelwert der Druck- und Biegezugfestigkeiten

- Standardabweichung der Druck- und Biegezugfestigkeit

- Meldung über Normkonformität der Prüfkörper hinsichtlich der geforderten Festigkeiten

Vorteile der Erfindung gegenüber dem genormten zerstörenden Verfahren der DIN EN 196-1:

- Herstellung eines Prismensatzes für den gesamten Prüfzeitraum

   ■ Zeitersparnis
     Herstellung einer geringeren Anzahl an Prismensätzen
   ■ Materialersparnis
     vor allem Reduzierung des Verbrauchs an Normensand
   ■ Geringere Prüfschwankungen durch Prüfung an gleichen Prismen über gesamten Zeitraum

- Messung zu beliebigen Zeitpunkten

   ■ Keine Berücksichtigung von Wocheenden und Feiertagen
   ■ Hohe Messwertdichte möglich
     Abbildung des gesamten Festigkeitsverlaufes

- Geringere Kapazitäten hinsichtlich der Lagerung der Prüfkörper notwendig

- Vorausberechnung der 28 Tage Druckfestigkeit

   ■ Ohne großen Aufwand leicht zu erzeugende Messwertdichte im Bereich von 1 bis 7 Tagen sorgt für geringen Erwartungsbereich des berechneten Wertes für die 28 Tage Festigkeit
   ■ Verbesserung der Kundenbetreuung über die Bauberatung

- Einsetzbar auch als Teillösung ohne die Automatisierung Ultraschallresonanzfrequenzmessung kann die Druckfestigkeits- und Biegezugfestigkeitsprüfung ersetzen

- Verwendbar auch für andere mineralische Bindemittel wie Kalk und Gips

Mit Hilfe dieses Prüfstandes ist es zudem möglich, bei eng gewählten Prüfterminen von 24 Stunden die Druckfestigkeit für das Alter von 28 Tagen voraus zu berechnen. Der Vorteil der zerstörungsfreien Prüfung kommt an dieser Stelle voll zu tragen, da trotz der erhöhten Anzahl an Prüfterminen alle Prüfungen am selben Prismensatz durchgeführt werden können.

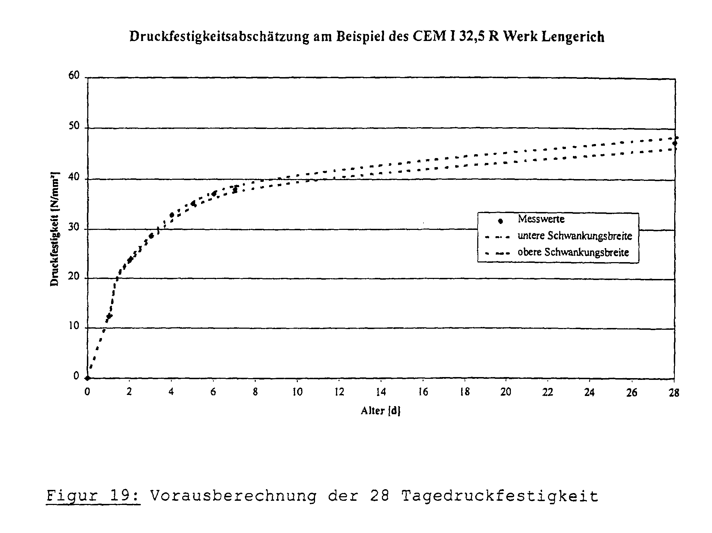

Figur 19 zeigt eine mögliche Vorausberechnung sowie die mit der Abschätzung verbundene Streuung des Wertes für die 28 Tagedruckfestigkeit.

Die Abschätzung ist anhand der nachstehenden Formel auf der Basis der 1, 2 und 7 Tageswerte der Druckfestigkeiten und den zugrundeliegenden Mittelwerten und Standardabweichungen durchgeführt worden.

Formel zur Abschätzung der 28 Tage Druckfestigkeit:

$$y = a - b \ln (x - c)$$

Die Variablen a, b und c wurden mit Hilfe des Programms Origin 6.0 G errechnet. Mit Hilfe der bestimmten Variablen ist der geschätzte Wert für die 28 Tagedruckfestigkeit berechnet worden. Die Variablen, die nur für das hier angeführte Beispiel gelten, wurden wie folgt ermittelt:

Tabelle 7:

| Variablen zur Berechnung der 28 Tage Druckfestigkeit für das gewählte Beispiel | |
| --- | --- |
| Variable | Wert |
| A | 23,34363 |

Tabelle 7:   (fortgesetzt)

| Variablen zur Berechnung der 28 Tage Druckfestigkeit für das gewählte Beispiel | |
| --- | --- |
| Variable | Wert |
| B | -7,10559 |
| C | -0,79217 |

Das Konvidenzintervall, gekennzeichnet durch den unteren und oberen Erwartungswert $\mu_u$ und $\mu_o$, wurde für einen Vertrauensbereich von 95 % und einer bekannten Standardabweichung der 28 Tage Druckfestigkeiten berechnet.

Formel zur Berechnung des Konvidenzintervalls

$$\left.\begin{array}{c} \mu_u \\ \mu_o \end{array}\right| \quad \bar{x} \;\pm\; 1{,}96 \quad s$$

Mit Hilfe des beschriebenen Verfahrens wurde der Wert für die 28 Tage Druckfestigkeit mit $47{,}2 \pm 1{,}1$ N/mm$^2$ berechnet. Mit einer Wahrscheinlichkeit von 95 % liegt der wahre Wert innerhalb des Bereiches von 46,1 bis 48,3 N/mm$^2$. Dieser Zement hatte einen Mittelwert und eine Standardabweichung von $46{,}4 \pm 0{,}6$ N/mm$^2$.

Nach Durchführung weiterer Korrelationsuntersuchungen und Untersuchung verschiedener Einflussgrößen auf die Resonanzfrequenz ist der Einsatz dieses Verfahren auch bei Variation des w/z-Wertes, den Zuschlägen, der Zusatzstoffe und Zusatzmittel denkbar. Die Erfassung und Charakterisierung der zuvor genannten Einflussgrößen ist die Voraussetzung, um dieses Verfahren auch in der Überwachung der Betonproduktion oder der Betonrezepturoptimierung einsetzen zu können.

Hierzu sind die nachstehenden Einflussgrößen und deren Einfluss auf die Resonanzfrequenzmessung zu charakterisieren:

- w/z-Wert

- Verdichtungsgrad

- Art der Zumahlstoffe (Hüttesand, Flugasche und Kalksteinmehl)

- Menge der Zumahlstoffe (Hüttesand, Flugasche und Kalksteinmehl)

- Zusatzmittel

  - Luftporenbildner
  - Verflüssiger
  - Beschleuniger
  - Verzögerer

- Zusatzstoffe im Beton

  - Flugasche
  - Mikrosilika
  - Gesteinsmehle

Ferner konnte festgestellt werden, dass es auch möglich ist, Festigkeitsänderungen aufgrund der damit verbundenen Gefügeveränderungen mit Hilfe der erfindungsgemäßen Frequenzanalyse zu charakterisieren. Bei auftretenden Festigkeitsänderungen verändert sich sowohl die Höhe der Resonanzfrequenz als auch die Dämpfung. Über eine Zerlegung des Frequenzspektrum in Ober- und Unterschwingungen können auftretende Mikrorisse durch eine Veränderung des Frequenzspektrums analysiert werden. Dadurch ist eine Aussage über den Grad der Schädigung und den damit verbundenen Auswirkungen verbunden.

Darüber hinaus sind mit der Erfindung weitere Vorteile erzielbar. Derzeit entfällt der größte Teil des Arbeitsaufwandes auf den Bereich der Formenreinigung. Nach dem Entformen der Prüfkörper und dem Zerlegen der Form muss jedes der insgesamt 7 Teile einzeln gereinigt und wieder zusammengebaut werden. Nach dem Zusammenbau erfolgt das erneute Abdichten. Dieser Herstellungsabschnitt kann durch die Verwendung einteiliger Kunststoffformen, insbesondere aus teflonisiertem Kunststoff oder Teflon vereinfacht werden.

Derzeit werden Stahlformen in der DIN EN 196 Teil 1 vorgeschrieben, das erfindungsgemäße Verfahren benötigt diese hohen Anforderungen hinsichtlich Planparallelität jedoch nicht. Um die Prüfkörper leichter Entformen zu können, werden erfindungsgemäß Kunststoffformen verwendet. Die Formen sind in den Abmessungen identisch zu den Stahlformen. In die Bodenplatte werden zum Ausblasen der Prüfkörper 2 konische Öffnungen je Prüfkörperteilform gefräst. Zum Entformen wird die Form mit der Öffnung nach unten gegen sechs an der Oberseite angebrachte Druckluftstutzen gepresst. Die Prüfkörper werden mittels Druckluft aus der Form gepresst.

Die Vorteile einer derartigen Form liegen in:

- Geringere Herstellungskosten ⇒ geringere Anschaffungskosten

- Geringeres Gewicht ⇒ leichteres Handling

- Schnelles Entformen der Prismen ⇒ Verringerung des Zeitaufwandes

Die Formenreinigung ist eng mit der Art und dem Material der Form verbunden. Bei Verwendung einer Kunststoffform entfällt das Abdichten der Form nach dem Zusammenbau. Die Reinigung im Vorfeld kann ebenfalls automatisiert werden. Die Reinigung der Form muss dazu in drei Teilabschnitte untergliedert werden. Im ersten Schritt sollte in einem Reinigungssystem die Reinigung mittels eines Wasserstrahls mit hohem Druck erfolgen. Der Druck des Wasserstrahls ist so zu wählen, dass er die Form nicht beschädigt, aber dennoch die an der Form haftenden Mörtelreste entfernen kann. Im zweiten Schritt erfolgt die Trocknung der Form. Vor dem Aufbringen eines dünnen Fettfilms im dritten Abschnitt erfolgt ein visuelle Kontrolle der Reinigung. Diese kann entfallen wenn Formen verwendet werden, die eine derart glatte und schlecht am Prüfkörper haftende Oberfläche aufweisen, daß die Entformung auch ohne Einfetten erfolgen kann. Dies ist beispielsweise bei Teflon- oder teflonisierten Formen der Fall.

## Patentansprüche

1. Verfahren zur zerstörungsfreien Bestimmung der Festigkeiten von seitlich sich verändernden Prüfkörpern, insbesondere Prüfprismen von nicht-metallisch anorganischen Werkstoffen, wie Baustoffen, Mörtel, Beton, Zement, Gips und dergleichen mittels der Resonanzfrequenzmethode, wobei die Festigkeit Y des Prüfkörpers nach der Formel

$$y = a\, e^{b\,x}$$

ermittelt wird, wobei

A) aus den physikalischen, ermittelten Größen Masse, Volumen und Resonanzfrequenz des Prüfkörpers der dynamische E-Modul X ermittelt wird;
B) als komplexe Eigenschaften der erhärtenden Bindemittel die Faktoren a und b ermittelt werden, wobei
a vom Lageparameter der RRSB-Verteilung sowie vom Steigungsmaß der RRSB-Verteilung und dem $C_3S$-Gehalt und dem $C_3A$-Gehalt sowie gegebenenfalls von Tracht und Habitus der Kristalle abhängt und
b von a als Startgröße abhängig ist und zudem vom $C_2S$-Gehalt sowie in geringem Maße vom $C_3S$- und $C_3A$-Gehalt sowie von Tracht und Habitus der Kristalle abhängt.

2. Verfahren nach Anspruch 1,
**dadurch gekennzeichnet, dass**
der Faktor a nach der Formel

$$a = 1{,}56607 - 0{,}158809 * x' + 0{,}766436 * n$$
$$+ 0{,}0352516 * C_3S + 0{,}110678 * C_3A$$

ermittelt wird.

**3.** Verfahren nach Anspruch 1 und/oder 2,
**dadurch gekennzeichnet, dass**
der Faktor b nach der Formel

$$b = 1{,}31478 * 10^{-4} - 1{,}10735 * 10^{-5} * a$$

$$- 6{,}2349 * 10^{-7} * x' - 2{,}15466 * 10^{-7} * C_3S$$

$$- 5{,}66977 * 10^{-7} * C_2S + 9{,}60419 * 10^{-7} * C_3A$$

ermittelt wird.

**4.** Verfahren nach einem oder mehreren der Ansprüche 1 bis 3, wobei

1. Prüfkörper angefertigt und automatisiert an einen individuellen Lagerort verbracht und dort unter gewünschten Bedingungen gelagert werden, wobei die Identifikation des Prüfkörpers über seine individualisierte Markierung und/oder den Lagerort erfolgt,
2. zu einem gewünschten Prüfzeitpunkt der Prüfkörper unter Identifizierung seiner Markierung dem Lagerort entnommen und die Prüfkörperabmessungen sowie die Prüfkörperrohdichte automatisiert ermittelt werden und in einer EDV gespeichert werden und
3. nach der Bestimmung der Masse und der Abmessungen die Bestimmung der Grundresonanzfrequenz automatisiert in einer weiteren Station erfolgt, wobei die Dehnungsresonanzfrequenz und auch die Torsionsresonanzfrequenz ermittelt werden und dem Prüfkörper zugeordnet in einer EDV gespeichert werden und die Festigkeiten aus den ermittelten Werten errechnet werden und anschließend der Prüfkörper automatisiert vorzugsweise seinem individuellen Lagerort wieder zugeführt wird.

**5.** Verfahren nach einem oder mehreren der Ansprüche 1 bis 4,
**dadurch gekennzeichnet, dass**
die Festigkeiten der Prüfkörper durch eine Ultraschallprüfung ermittelt werden.

**6.** Verfahren nach einem oder mehreren der Ansprüche 1 bis 5,
**dadurch gekennzeichnet, dass**
die Prüfkörper bei Unterwasser-Lagerung vor der Bestimmung der Masse automatisiert von anhaftendem Wasser befreit werden.

**7.** Verfahren nach Anspruch 6,
**dadurch gekennzeichnet, dass**
die Prüfkörper bei Unterwasser-Lagerung vor der Bestimmung der Masse durch Druckluftstöße und/oder durch Neigen und/oder Drehen von anhaftendem Wasser befreit werden.

**8.** Verfahren nach einem oder mehreren der vorhergehenden Ansprüche,
**dadurch gekennzeichnet, dass**
die Prüfkörperabmessungen mit Hilfe eines Lasersystems und/oder über induktive Wegaufnehmer ermittelt werden.

**9.** Verfahren nach einem oder mehreren der vorhergehenden Ansprüche,
**dadurch gekennzeichnet, dass**
die Prüfköpfe zur Ermittlung der Resonanzfrequenzen mit einem Ankopplungsdruck von 0,7 bis 1,3 N/mm$^2$ an die Prüfkörper angekoppelt werden.

**10.** Verfahren nach einem oder mehreren der vorhergehenden Ansprüche,
**dadurch gekennzeichnet, dass**
die Prüfkörper mit Hilfe einer automatisch auslesbaren Kennzeichnung und über die Lage der Prüfkörper an ihrem Lagerort identifiziert werden.

**11.** Verfahren nach einem oder mehreren der vorhergehenden Ansprüche,
**dadurch gekennzeichnet, dass**
die Einlagerung der Prüfkörper über ein Robotersystem erfolgt, welches auf einer Linearachse die Front der Lagerorte abfahren kann, wobei mit Hilfe eines Robotergreifarms die Breite (Y-Achse) und Tiefe (Z-Achse) des Lagerorts erreicht werden kann, wobei nach der Einlagerung die Koordinaten des Lagerplatzes mit der Prüfkörperkennzeichnung gemeinsam gekoppelt zur späteren Identifikation gespeichert werden.

**12.** Verfahren nach einem oder mehreren der vorhergehenden Ansprüche,
**dadurch gekennzeichnet, dass**
die Prüfkörper in Wasserbecken gelagert werden.

**13.** Verfahren nach einem oder mehreren der vorhergehenden Ansprüche,
**dadurch gekennzeichnet, dass**
die Prüfkörper zur Einlagerung und Entnahme vom Roboter mit einem Robotergreifarm gegriffen werden, wobei am Robotergreifarm Mittel zur Identifikation der Kennzeichnung des Prüfkörpers vorgesehen sind.

**14.** Verfahren nach einem oder mehreren der vorhergehenden Ansprüche,
**dadurch gekennzeichnet, dass**
zur Kennzeichnung und Identifikation der Prüfkörper eine Kombination aus RFID, einer Ink-Jet-Kennzeichnung und der Lage im Wasserbecken oder von RFID, bedruckten und auf dem Prüfkörper angeordneten Etiketten und der Lage im Wasserbecken oder von RFID und der Lage im Wasserbecken angewandt wird.

**15.** Verfahren nach Anspruch 14,
**dadurch gekennzeichnet, dass** zur Speicherung der im Prüfungszeitraum ermittelten Daten durch RFID ein externer Computer verwendet wird.

**16.** Verfahren nach Anspruch 15,
**dadurch gekennzeichnet, dass**
der RFID-Datenträger durch ein Butyl-Dichtungsband mit dem Prüfkörper verbunden ist.

**17.** Verfahren nach Anspruch 16,
**dadurch gekennzeichnet, dass**
das Butyl-Dichtungsband nach dessen Aufbringung auf den Prüfkörper erwärmt wird, bevor der Datenträger aufgedrückt wird.

**18.** Verfahren nach Anspruch 14,
**dadurch gekennzeichnet, dass**
für die Identifikation nach der Lage der Prismen im Wasserbecken die Wasserbecken in einzelne Lageplätze aufgeteilt und mit XY-Koordinaten versehen werden und Kunststoffprofile in den Becken angebracht werden.

**19.** Verfahren nach Anspruch 18,
**dadurch gekennzeichnet, dass**
als Kunststoffprofil ein flacher Gitterkorb verwendet wird.

**20.** Vorrichtung zur zerstörungsfreien Bestimmung der Festigkeiten von Prüfkörpern, insbesondere zum Durchführen des Verfahrens nach einem oder mehreren der Ansprüche 1 bis 19,
**gekennzeichnet durch**

- zumindest einen die Prüfkörper individualisierend unter vorher bestimmten Bedingungen lagernden Lagerort,
- eine Entnahme und Versetzeinrichtung für die Prüfkörper, insbesondere ein verfahrbarer Roboter mit Greifarm,
- einen Zwischenlagerplatz am verfahrbaren Robotersystem (2) zum Zwischenlagern von zumindest drei Prüfkörpern eines Prüftermins,
- Messplätze (7, 8, 9) sowie davor angeordnete Lagerplätze (5, 10), wobei zudem eine Messstelle zur Bestimmung der Masse vorgesehen ist, eine Messstelle (8) zur Bestimmung der Prüfkörperdimension und eine Messstelle (9) zur Prüfung der Grundresonanzen vorgesehen ist, wobei ein weiterer die Prüfkörper, welche vom Zwischenlagerplatz (4) mit dem Roboter (2) zu den Lagerplätzen (5) verfahren werden, den drei Messplätzen zuführender und nach dem erfolgten Messen auf dem zweiten Profil (10) ablegender Roboter (6) vorhanden ist,
- eine die Prüfkörperabmessungen sowie die Prüfkörperrohdichte speichernde und nach Messung und Verifizierung aller Daten die Berechnung der Festigkeit nach einer Exponentialfunktion der allgemeinen Formel

$$y = a\, e^{b\,x}$$

vornehmende den dynamischen E-Moduls x aus den physikalischen, ermittelten Größen Masse, Volumen und Resonanzfrequenz ermittelnde und die Faktoren a und b als komplexe Eigenschaften der erhärtenden Bindemittel,

wobei a vom Lageparameter der RRSB-Verteilung sowie vom Steigungsmaß der RRSB-Verteilung und dem $C_3S$-Gehalt und dem $C_3A$-Gehalt sowie gegebenenfalls von Tracht und Habitus der Kristalle abhängt, und b von a als Startgröße abhängig ist und zudem vom $C_2S$-Gehalt sowie in geringem Maße vom $C_3S$- und $C_3A$-Gehalt sowie von Tracht und Habitus der Kristalle abhängt, ermittelnde EDV.

**21.** Vorrichtung nach Anspruch 20,
**dadurch gekennzeichnet, dass**
sie einen verfahrbaren Roboter mit Greifarm für die Entnahme und Versetzeinrichtung aufweist.

**22.** Vorrichtung nach Anspruch 20,
**dadurch gekennzeichnet, dass**
eine EDV zur Speicherung der Messwerte vorhanden ist.

**23.** Vorrichtung nach Anspruch 20 und/oder 22,
**dadurch gekennzeichnet, dass**
der oder die Roboterarme, welche die Prüfkörper greifen, Einrichtungen zum Erkennen und Auslesen der Prüfkörperkennzeichnung aufweisen.

**Claims**

**1.** Method for non-destructive determination of the strengths of laterally changing test pieces, in particular test prisms of non-metallic inorganic materials, such as building materials, mortar, concrete, cement, gypsum and the like, by means of the resonant frequency method, the strength Y of the test piece being determined on the basis of the formula

$$y = a\, e^{b\,x}$$

where

A) the dynamic modulus of elasticity X is determined from the physical, determined variables of mass, volume and resonant frequency of the test piece;

B) the factors a and b are determined as complex properties of the hardening binder, where
    a is dependent on the positional parameters of the RRSB distribution and on the degree of slope of the RRSB distribution and the $C_3S$ content and the $C_3A$ content and also possibly on the tracht and habit of the crystals, and
    b is dependent on a as a starting value and moreover a is dependent on the $C_2S$ content and to a lesser extent on the $C_3S$ and $C_3A$ content and also on the tracht and habit of the crystals.

**2.** Method according to Claim 1, **characterized in that** the factor a is determined on the basis of the formula

$$a = 1{,}56607 - 0{,}158809 * x' + 0{,}766436 * n$$

$$+ 0{,}0352516 * C_3S + 0{,}110678 * C_3A$$

**3.** Method according to Claim 1 and/or 2, **characterized in that** the factor b is determined on the basis of the formula

$$b = 1,31478 * 10^{-4} - 1,10735 * 10^{-5} * a$$

$$- 6,2349 * 10^{-7} * x' - 2,15466 * 10^{-7} * C_3S$$

$$- 5,66977 * 10^{-7} * C_2S + 9, 60419 * 10^{-7} * C_3A$$

4. Method according to one or more of Claims 1 to 3, where

    1. test pieces are prepared and automatically brought to an individual storage location and stored there under desired conditions, the identification of the test piece taking place by means of its individualized marking and/or the storage location,

    2. at the desired testing time, the test piece is removed from the storage location, with its marking being identified, the dimensions of the test piece and the apparent density of the test piece are automatically determined and stored in an EDP unit and

    3. following the determination of the mass and the dimensions, the determination of the fundamental resonant frequency takes place automatically in a further station, the extensional resonant frequency and also the torsional resonant frequency being determined and stored, assigned to the test piece, in an EDP unit, and the strengths are calculated from the determined values and the test piece is subsequently automatically fed back again preferably to its individual storage location.

5. Method according to one or more of Claims 1 to 4, **characterized in that** the strengths of the test pieces are determined by an ultrasonic test.

6. Method according to one or more of Claims 1 to 5, **characterized in that**, in the case of underwater storage, the test pieces are automatically freed of adherent water before the determination of the mass.

7. Method according to Claim 6, **characterized in that**, in the case of underwater storage, the test pieces are freed of adherent water before the determination of the mass by blasts of compressed air and/or by tilting and/or turning.

8. Method according to one or more of the preceding claims, **characterized in that** the dimensions of the test pieces are determined with the aid of a laser system and/or by means of inductive displacement pickups.

9. Method according to one or more of the preceding claims, **characterized in that**, to determine the resonant frequencies, the test heads are coupled to the test pieces with a coupling pressure of 0.7 to 1.3 N/mm$^2$.

10. Method according to one or more of the preceding claims, **characterized in that** the test pieces are identified with the aid of an automatically readable marking and by means of the position of the test pieces at their storage location.

11. Method according to one or more of the preceding claims, **characterized in that** the storing of the test pieces takes place by means of a robot system which can travel to the front of the storage locations on a linear axis, it being possible for the width (Y axis) and depth (Z axis) of the storage location to be reached with the aid of a robot gripping arm, the coordinates of the storage place being stored after storage, coupled together with the test piece marking for later identification.

12. Method according to one or more of the preceding claims, **characterized in that** the test pieces are stored in water tanks.

13. Method according to one or more of the preceding claims, **characterized in that**, for storage and removal, the test pieces are gripped by the robot with a robot gripping arm, means for identifying the marking of the test piece being provided on the robot gripping arm.

14. Method according to one or more of the preceding claims, **characterized in that** a combination of RFID, an ink-jet marking and the position in the water tank or of RFID, labels bearing printing and arranged on the test piece and the position in the water tank or of RFID and the position in the water tank is used for the marking and identification of the test pieces.

15. Method according to Claim 14, **characterized in that** an external computer is used for storing by RFID the data determined in the test period.

16. Method according to Claim 15, **characterized in that** the RFID data carrier is connected to the test piece by a butyl sealing strip.

17. Method according to Claim 16, **characterized in that**, after it has been applied to the test piece, the butyl sealing strip is heated before the data carrier is pressed on.

18. Method according to Claim 14, **characterized in that**, for the identification on the basis of the position of the prisms in the water tank, the water tanks are divided into individual storage places and provided with XY coordinates and plastic profiles are fitted in the tanks.

19. Method according to Claim 18, **characterized in that** a flat slatted cage is used as the plastic profile.

20. Apparatus for non-destructive determination of the strengths of test pieces, in particular for carrying out the method according to one or more of Claims 1 to 19, **characterized by**

   - at least one storage location storing the test pieces in an individualizing manner under previously determined conditions,
   - a removing and displacing device for the test pieces, in particular a movable robot with a gripping arm,
   - an intermediate storage place on the movable robot system (2) for intermediately storing at least three test pieces of one test session,
   - measuring places (7, 8, 9) and storage places (5, 10) arranged in front of them, a measuring location for determining the mass also being provided, a measuring location (8) for determining the dimensions of the test pieces and a measuring location (9) for testing the fundamental resonances being provided, a further robot (6) being present, supplying the three measuring places with the test pieces which are moved by the robot (2) from the intermediate storage location (4) to the storage places (5) and setting them down on the second profile (10) once measuring has taken place,
   - an EDP unit, storing the dimensions of the test piece and the apparent density of the test piece and, after measurement and verification of all the data, performing the calculation of the strength on the basis of an exponential function of the general formula

$$y = a\,e^{b\,x}$$

   determining the dynamic modulus of elasticity X from the physical, determined variables of mass, volume and resonant frequency and determining the factors a and b as complex properties of the hardening binder, where a is dependent on the positional parameters of the RRSB distribution and on the degree of slope of the RRSB distribution and the $C_3S$ content and the $C_3A$ content and also possibly on the tracht and habit of the crystals, and b is dependent on a as a starting value and moreover a is dependent on the $C_2S$ content and to a lesser extent on the $C_3S$ and $C_3A$ content and also on the tracht and habit of the crystals.

21. Apparatus according to Claim 20, **characterized in that** it has a movable robot with a gripping arm for the removing and displacing device.

22. Apparatus according to Claim 20, **characterized in that** an EDP unit is present for storing the measured values.

23. Apparatus according to Claims 20 and/or 22, **characterized in that** the robot arm or arms which grip the test pieces have devices for detecting and reading the test piece marking.

**Revendications**

1. Procédé pour la détermination non destructive des résistances mécaniques d'éprouvettes qui varient latéralement, en particulier de prismes d'essai de matériaux non métalliques inorganiques tels que les matériaux de construction, le mortier, le béton, le ciment, le plâtre et analogues, par la méthode de la fréquence de résonance, la résistance mécanique Y de l'éprouvette étant calculée en application de la formule :

$$y = a\,e^{\,b\,x}$$

où

A) le module E dynamique X est calculé à partir des grandeurs physiques constatées, masse, volume et fréquence de résonance de l'éprouvette ;

B) les facteurs $\underline{a}$ et $\underline{b}$ sont obtenus en tant que propriétés complexes du liant durcissant, où

$\underline{a}$ dépend du paramètre de position de la distribution RRSB ainsi que de la pente de la distribution RRSB, et de la teneur en $C_3S$ et de la teneur en $C_3A$, ainsi qu'éventuellement du nombre de faces et de la forme des faces des cristaux et

$\underline{b}$ dépend de $\underline{a}$ en tant que grandeur de départ et, en supplément de la teneur en $C_2S$ ainsi que, à un degré réduit, de la teneur en $C_3S$ et en $C_3A$, ainsi que du nombre de faces et de la forme des faces des cristaux.

2. Procédé selon la revendication 1, **caractérisé en ce que** le facteur $\underline{a}$ est calculé en application de la formule

$$a = 1{,}56607 - 0{,}158809 * x' + 0{,}766436 * n$$
$$+ 0{,}0352516 * C_3S + 0{,}110678 * C_3A$$

3. Procédé selon la revendication 1 et/ou 2, **caractérisé en ce que** le facteur b est calculé en application de la formule

$$b = 1{,}31478 * 10^{-4} - 1{,}10735 * 10^{-5} * a$$
$$- 6{,}2349 * 10^{-7} * x' - 2{,}15466 * 10^{-7} * C_3S$$
$$- 5{,}66977 * 10^{-7} * C_2S + 9{,}60419 * 10^{-7} * C_3A$$

4. Procédé selon une ou plusieurs des revendications 1 à 3, dans lequel

1) des éprouvettes sont préparées et placées à un lieu d'entreposage individuel de façon automatisée et, là, entreposées dans des conditions souhaitées, l'identification de l'éprouvette s'effectuant au moyen de son marquage individualisé et/ou au moyen du lieu d'entreposage.

2) au moment souhaité de l'essai, l'éprouvette est prise sur le lieu d'entreposage sous identification de son marquage, et les dimensions de l'éprouvette ainsi que la masse volumique apparente de l'éprouvette sont obtenues de façon automatisée et mémorisées dans un dispositif informatique.

3) après la détermination de la masse et des dimensions, la détermination de la fréquence de résonance de base s'effectue de façon automatisée dans une autre station dans laquelle la fréquence de résonance de dilatation et aussi la fréquence de résonance de torsion sont elles aussi obtenues et associées à l'éprouvette et mémorisées dans un dispositif informatique et les résistances sont calculées à partir des valeurs obtenues, puis l'éprouvette est renvoyée de préférence à son lieu d'entreposage individuel, de façon automatisée.

5. Procédé selon une ou plusieurs des revendications 1 à 4, **caractérisé en ce que** les résistances mécaniques des éprouvettes sont obtenues par un essai aux ultra-sons.

6. Procédé selon une ou plusieurs des revendications 1 à 5, **caractérisé en ce que**, dans le cas d'un entreposage sous l'eau, les éprouvettes sont débarrassées de l'eau qui y adhère de façon automatisée avant la détermination de la masse.

7. Procédé selon la revendication 6, **caractérisé en ce que**, dans le cas d'un entreposage sous l'eau, les éprouvettes sont débarrassées de l'eau qui y adhère avant la détermination de la masse par des coups d'air comprimé et/ou par inclinaison et rotation.

8. Procédé selon une ou plusieurs des revendications précédentes, **caractérisé en ce que** les dimensions des éprouvettes sont mesurées à l'aide d'un système laser et/ou au moyen d'un capteur de déplacement inductif.

**9.** Procédé selon une ou plusieurs des revendications précédentes, **caractérisé en ce que**, pour la détermination des fréquences de résonance, les têtes d'essai sont accouplées aux éprouvettes avec une pression d'accouplement de 0,7 à 1,3 N/mm$^2$.

**10.** Procédé selon une ou plusieurs des revendications précédentes, **caractérisé en ce que** les éprouvettes sont identifiées sur leur lieu d'entreposage à l'aide d'un marquage pouvant être lu de façon automatique et par la position des éprouvettes.

**11.** Procédé selon une ou plusieurs des revendications précédentes, **caractérisé en ce que** l'entreposage des éprouvettes est exécuté au moyen d'un système robotisé qui peut parcourir la surface frontale des lieux d'entreposage des éprouvettes sur un axe linéaire, la largeur (axe Y) et la profondeur (axe Z) du lieu d'entreposage peuvent être atteintes à l'aide d'un bras de préhension de robot et, après la mise en entreposage, les coordonnées de l'emplacement d'entreposage sont mémorisées, couplées en commun avec le marquage de l'éprouvette en vue de l'identification ultérieure.

**12.** Procédé selon une ou plusieurs des revendications précédentes, **caractérisé en ce que** les éprouvettes sont entreposées dans des bassins d'eau.

**13.** Procédé selon une ou plusieurs des revendications précédentes, **caractérisé en ce que** pour la mise en entreposage et le prélèvement, les éprouvettes sont saisies par des robots possédant un bras de préhension de robot, des moyens pour l'identification du marquage de l'éprouvette étant prévus sur le bras de préhension du robot.

**14.** Procédé selon une ou plusieurs des revendications précédentes, **caractérisé en ce que**, pour le marquage et l'identification des éprouvettes, on utilise une combinaison de RFID, d'un marquage à jet d'encre et de la position dans le bassin d'eau, ou encore d'étiquettes RFID imprimées et disposées sur l'éprouvette, et de la position dans le bassin d'eau, ou de RFID et de la position dans le bassin d'eau.

**15.** Procédé selon la revendication 14, **caractérisé en ce que**, pour la mémorisation des données obtenues pendant la durée de l'essai, on utilise un ordinateur externe.

**16.** Procédé selon la revendication 15, **caractérisé en ce que** le support des données RFID est relié à l'éprouvette par un ruban d'étanchéité en butyle.

**17.** Procédé selon la revendication 16, **caractérisé en ce que** le ruban d'étanchéité en butyle est chauffé après son application sur l'éprouvette, avant que le support de données ne soit imprimé.

**18.** Procédé selon la revendication 14, **caractérisé en ce que** pour l'identification d'après la position des prismes dans le bassin d'eau, les bassins d'eau sont divisé en emplacements d'entreposage distincts et munis de coordonné XY, et des profilés en matière plastique sont mis en place dans les bassins.

**19.** Procédé selon la revendication 18, **caractérisé en ce que**, comme profilé en matière plastique, on utilise un panier plat en grillage.

**20.** Dispositif pour la détermination non destructive des résistances mécaniques d'éprouvettes, en particulier pour la mise en oeuvre du procédé selon une ou plusieurs des revendications 1 à 19, **caractérisé par**

- au moins un lieu d'entreposage qui entrepose les éprouvettes en les individualisant, dans des conditions déterminées à l'avance,
- un dispositif de prélèvement et de déplacement pour les éprouvettes, en particulier un robot mobile possédant un bras de préhension,
- un emplacement d'entreposage intermédiaire sur le système de robot mobile (2) pour l'entreposage intermédiaire d'au moins trois éprouvettes d'une période d'essai,
- des emplacements de mesure (7, 8, 9) ainsi que des emplacements d'entreposage (5, 10) disposés en avant de ceux-ci, cependant qu'il est prévu en supplément une zone de mesure pour la détermination de la masse, une zone de mesure (8) pour la détermination de la dimension de l'éprouvette et une zone de mesure (9) pour le contrôle des résonances de base, et qu'il est prévu un autre robot (6) qui amène aux trois emplacements de mesure les éprouvettes qui sont amenées de l'emplacement d'entreposage intermédiaire (4) aux emplacements d'entreposage (5) au moyen du robot (2) et, après exécution de la mesure, les dépose sur le deuxième profilé (10),

- un dispositif informatique qui mémorise les dimensions des éprouvettes ainsi que la masse volumique apparente des éprouvettes et, après la mesure et la vérification de toutes les données, effectue le calcul de la résistance mécanique en application d'une fonction exponentielle de la formule générale :

$$y = a\,e^{b\,x}$$

calcule le module E dynamique x à partir des grandeurs physiques, masse, volume et fréquence de résonance obtenus, et calcule les facteurs $\underline{a}$ et $\underline{b}$ en tant que propriétés complexes du liant durcissant, où $\underline{a}$ dépend du paramètre de position de la distribution RRSB ainsi que de la pente de la distribution RRSB, et de la teneur en $C_3S$ et de la teneur en $C_3A$, ainsi qu'éventuellement du nombre de faces et de la forme des faces des cristaux et

$\underline{b}$ dépend de $\underline{a}$ en tant que grandeur de départ et, en supplément de la teneur en $C_2S$ ainsi que, à un degré réduit, de la teneur en $C_3S$ et en $C_3A$, ainsi que du nombre de faces et de la forme des faces des cristaux.

21. Dispositif selon la revendication 20, **caractérisé en ce qu'**il comprend un robot mobile possédant un bras de préhension pour l'enlèvement et un dispositif de déplacement.

22. Dispositif selon la revendication 20, **caractérisé en ce qu'**il est prévu un dispositif informatique pour la mémorisation des valeurs de mesure.

23. Dispositif selon la revendication 20 et/ou 22, **caractérisé en ce que** le ou les bras de robot qui saisissent les éprouvettes présentent des dispositifs destinés à reconnaître et lire le marquage des éprouvettes.

.125 / 3,175
.625 / .16
.920 / 23.4
.225 / 4.7
ES620
inches / mm

Figur 1: RFID-System (Lesesystem mit passivem Datenträger)

Figur 2: Abtropfkurven von Mörtelprismen unterschiedlicher Neigung und Drehung in Abhängigkeit der Zeit

30

<u>Figur 3:</u>  Seitenansicht Prisma mit Meßköpfen

**Graphische Darstellung des Mittelwertes als Funktion der Anzahl der Messung n**

<u>Figur 4:</u>  Veränderungen        der        Mittelwerte        und

Standardabweichungen  durch  wiederholtes  Messen  als

Funktion  der  Anzahl  n

**Verlauf der Verstärkung des Messsignales und der ermittelten Resonanzfrequenz in Abhängigkeit der Anpresskraft der Meßköpfe**

Verstärkung des Meßsignales [%]

Resonanzfrequenz [Hz]

● Verstärkung
■ Ermittelte Resonanzfrequenz

Anpreßkraft [N]

Figur 5: Verstärkung V und Resonanzfrequenz $n_D$ in Abhängigkeit der Anpresskraft

Verhältnis $n_D : n_T$

● CEM I 32,5 R Deuna
■ CEM I 42,5 R Deuna
▲ CEM I 52,5 R Geseke

Probenalter [h]

Figur 6: Verhältnis der Grundresonanzen zueinander in Abhängigkeit des Probenalters

**CEM I 32,5 R im Vergleich verschiedener Werke der Dyckerhoff Gruppe**

Figur 7: Korrelation des dynamischen E-Moduls und der Druckfestigkeit am Beispiel des CEM I 32,5 R verschiedener Werke

$$y = a \, x^b$$

Figur 8: Fittfunktion Typ Allomeric

**Figur 9:** Korrelation zerstörungsfrei und zerstörend bestimmter Druck- und Biegezugfestigkeiten am Beispiel des CEM I 32,5 R des Werkes IV

**Figur 10:** Übersicht der Bestimmtheitsmaße aus Druck- und Biegezugfestigkeitskorrelation

Figur 11: Faktor a als Funktion des Lageparameters x'

Korrelation des bestimmten Faktors a mit dem berechneten Faktor a

$$y = 0,989x$$
$$R^2 = 0,8032$$

Figur 12: Korrelation des berechneten Faktor mit dem bestimmten Faktor a

**Exponent b als Funktion des Faktor a**

Figur 13: Exponent b als Funktion des Faktors a

**Korrelation des bestimmten mit dem errechneten Exponenten b**

Figur 14: Korrelation zwischen dem berechneten Exponenten und dem bestimmten Exponenten b

Fig.15 : Blockschaltbild der Prüfstandsapparatur

Figur 16: Schematische Darstellung des Robotersystems und Anordnung der Messstellen

Fig. 17 : Einspannvorrichtung (DIN 51 915) zur Bestimmung des E-Moduls

Prüfkörperauflage an den Schwingungsknoten der ersten Oberwelle [6]

Fig. 18 : Einspannvorrichtung zur Bestimmung der Torsionfrequenz [6]

**Druckfestigkeitsabschätzung am Beispiel des CEM I 32,5 R Werk Lengerich**

Figur 19: Vorausberechnung der 28 Tagedruckfestigkeit